# EUROPEAN PATENT APPLICATION

(11) **EP 2 184 068 A1**
(43) Date of publication of application: **12.05.2010**
(21) Application number: 08805370.7
(22) Date of filing: 04.08.2008
(51) Int. Cl.: A61K 35/34, C12N 5/08

(54) **POPULATION OF ADULT STEM CELLS DERIVED FROM CARDIAC ADIPOSE TISSUE AND USE THEREOF IN CARDIAC REGENERATION**

(30) Priority: 03.08.2007 ES 200702205
(71) Applicant: Genetrix, S.L., 28760 Madrid (Tres Cantos) (ES); Fundació Privada Institut De Recerca De L'hospital De La Santa Creu I Sant Pau, 08025 Barcelona (ES)
(72) Inventor: BÜSCHER, Dirk, E-28760 Madrid (tres Cantos) (ES); BAYES GENIS, Antonio, E-08025 Barcelona (ES); ROURA FERRER, Santiago, E-08025 Barcelona (ES); FARRÉ CRESPO, Jordi, E-08025 Barcelona (ES); PRAT VIDAL, Cristina, E-08025 Barcelona (ES)
(74) Representative: ABG Patentes, S.L.
(86) International application number: PCT/ES2008/000543
(87) International publication number: WO 2009/027563

(57) **Abstract**

The present invention relates to the isolation and characterization of a novel population of adult stem cells derived from fatty heart tissue, which constitutively express GATA-4 and/or Cx43. Said cell population can be used in cell therapy protocols in order to regenerate damaged myocardial tissue.

## Description

### Field of the Invention

The present invention relates to the isolation and characterization of a novel population of adult stem cells derived from fatty heart tissue and to its potential therapeutic applications. Specifically, the invention relates to the use of said population of adult stem cells derived from fatty heart tissue in cell therapy protocols in order to regenerate damaged myocardial tissue.

### Background of the Invention

The huge social and economic impact that degenerative diseases have today in developed countries, including cardiovascular pathologies, has promoted the search for cell precursors that can improve conventional therapies.

The main consequence of a myocardial infarction is the irreversible loss of a portion of the heart muscle which is replaced by scar tissue. This loss causes a reduction of the contractile capability of the myocardium as well as of the pumping function to provide the necessary cardiac output, overloading the surviving myocardium and ultimately leading to heart failure. In 1998, in the United States alone there were more than 7.5 million people who survived a myocardial infarction. Of these survivors, more than 30% die during the first year after the infarction. Survival after infarction largely depends on the size of the dead myocardium area due to a lack of vascularization. In humans, an infarction of more than 45% of the mass of the left ventricle causes irreversible cardiogenic shock.

This local loss of myocardium causes the reorganization of the rest of the heart muscle, with increased cell death due to apoptosis, hypertrophy of the muscle cells and increased fibrosis in the surviving myocardium. This reorganization of the heart muscle, commonly known as "remodeling", very frequently results in the onset of heart failure. In this situation, the heart is unable to maintain a suitable cardiac output, resulting in a serious and progressive limitation of the individual's capability.

After an acute myocardial infarction, as well as in those patients who have developed congestive heart failure, current therapy is unable to fully recover the myocardium of these patients with severe refractory ventricular dysfunction. All the therapies used today to treat the loss of heart muscle are aimed at preserving the function of the remaining myocardium.

The objective of said therapies is to preserve and improve the function of cardiomyocytes (the contractile cells of the heart) that have survived and to prevent their death either due to apoptosis or necrosis. Most treatments for myocardial infarction attempt to restore the blood flow to the ischemic area to prevent the loss of more contractile cells. These reperfusion therapies include the use of thrombolytic agents (which dissolve the thrombus formed in the coronary artery), balloon angioplasty (to open the closed artery by physical methods) or coronary bypass in which the closed area is surpassed by means of a bridge joining the proximal part with the distal part of the with the part distal of the obstructed artery by means of a vein graft. In 1998 alone in United States, more than half a million balloon angioplasties and a similar number of bypass surgeries were performed. These treatments frequently achieve re-establishing the coronary flow to the damaged area and avoid for a certain time the additional loss of heart muscle. However, none of them are able to recover the tissue that is already dead at the time of the intervention. If this loss is substantial, the long-term consequence is the development of chronic heart failure which progresses into terminal heart failure.

Until now, the only option for the treatment of terminal heart failure which allows completely recovering the cardiac function is a heart transplant. However, organ transplanting presents a number of complications such as the scarcity of donors, the high probability of rejection of the transplanted organ, etc.

In the particular case of heart failure, due to the scarcity of donors, a transplant is an option that is available to a maximum of 5% of the patients in need thereof. Assuming that the high cost of the intervention is not a sufficiently limiting obstacle, the high cost of life-long immunosuppressive therapy and the high number of neoplasias these patients suffer as a result are other limitations of this therapeutic modality.

It was traditionally considered that cardiomyocytes only divided during embryonic-fetal development, being irreversibly lost after a myocardial infarction, such that until a relatively short time ago, cardiac biology experts considered that the heart did not have self-regenerative potential. In light of recent studies, this concept has changed radically.

Cell therapy or cardiomyoplasty, based on the principle of the regenerative capability of the heart from the application of stem cells, is discerned as a promising therapeutic route in the treatment of the heart diseases. In the last five years a series of studies have been conducted that has clearly shown the processes of myocardial regeneration, demonstrating the presence of resident stem cells and/or extracardiac stem cells with regenerative potential in adult hearts. In this context, the observation of post-transplant cardiac chimerism was a reliable test of the existence of cells capable of colonizing the myocardial tissue and adopting a cardiac fate [Bayés-Genís A. et al., 2002, Host-cell derived cardiomyocytes in Sex-mismatch cardiac allografts. Cardiovasc Res 2002; 404-410].

One of the main objectives sought by research in this field is to identify the optimal type of stem cell that can be safely and effectively applied in cardiac regeneration therapies. The suitable type of stem cell for heart regeneration must be capable of expanding sufficiently, showing a capability for differentiation of a fully functional cardiomyocyte, being integrated in the myocardial tissue establishing electrochemical contact with the adjacent cells, and finally, its application not being limited by issues of immunologic rejection or ethical considerations.

Stem cells are characterized by their self-maintenance capability and by their plasticity, this latter term relating to their capability to differentiate into one or more cell lineages under the suitable stimuli. Stem cells are basically classified according to criteria of cell lineage or strain, organ or tissue of origin; expression of specific surface markers, expression of transcription factors and/or proteins; and capability for differentiation, i.e., number and type of specialized cells which may be generated.

Among stem cells, there is a clear distinction between those stem cells that can be obtained during one of the first stages of the development of an embryo (blastocyte), known as embryonic stem cells, and those that come from adult somatic tissues, referred to as adult stem cells. An adult stem cell is a non-differentiated cell found in a differentiated tissue and has the capability for proliferation and differentiation into one or more cell types. Adult stem cells are present in various adult tissues, their presence in bone marrow, adipose tissue, blood, cornea, retina, brain, skeletal muscle, dental pulp, gastrointestinal epithelium, liver and skin being broadly described. Due to their nature, autologous adult stem cells are immunocompatible and the use thereof presents no ethical problems.

Despite having identified resident cardiac stem cells [Beltrami, A.P. et al. Adult cardiac stem cells are multipotent and support myocardial regeneration. Cell 114, 763-76 (2003); Oh, H. et al. Cardiac progenitor cells from adult myocardium: homing, differentiation, and fusion after infarction. Proc Natl Acad Sci U S A 100, 12313-8 (2003)], tissue repair after damage is deficient and populations of alternative adult non-cardiac stem cells have been investigated [Goldstein, G. et al. Human umbilical cord blood biology, transplantation and plasticity. Curr Med Chem 13, 1249-59 (2006); Orlic, D. et al. Transplanted adult bone marrow cells repair myocardial infarcts in mice. Ann N Y Acad Sci 938, 221-9; discussion 229-30 (2001); Pittenger, M.F. & Martin, B.J. Mesenchymal stem cells and their potential as cardiac therapeutics. Circ Res 95, 9-20 (2004); Rangappa, S., Fen, C., Lee, E.H., Bongso, A. & Sim, E.K. Transformation of adult mesenchymal stem cells isolated from the fatty tissue into cardiomyocytes. Ann Thorac Surg 75, 775-9 (2003); Zvaifler, N.J. et al. Mesenchymal precursor cells in the blood of normal individuals. Arthritis Res 2, 477-88 (2000)].

Adult stem cells have been tested in a variety of mammal hearts after injury, from mice to human beings. In mice, an enormous expectation came about with bone marrow-derived stem cells, although such expectation has been attenuated by reports showing that their potential for cardiac regeneration is limited and controversial [Balsam, L.B. et al. Haematopoietic stem cells adopt mature haematopoietic fates in ischaemic myocardium. Nature 428, 668-73 (2004); Murry, C.E. et al. Haematopoietic stem cells do not transdifferentiate into cardiac myocytes in myocardial infarcts. Nature 428, 664-8 (2004)]. In human beings, although certain improvement in the cardiac function in preliminary clinical studies was shown [Assmus, B. et al. Transplantation of Progenitor Cells and Regeneration Enhancement in Acute Myocardial Infarction (TOPCARE-AMI). Circulation 106, 3009-17 (2002); Strauer, B.E. et al. Repair of infarcted myocardium by autologous intracoronary mononuclear bone marrow cell transplantation in humans. Circulation 106, 1913-8 (2002)], more recent clinical trials only show a moderate increase in the cardiac function after supplying cells [Dohmann, H.F. et al. Multicenter Double Blind Trial of Autologous Bone Marrow Mononuclear Cell Transplantation through Intracoronary Injection post Acute Myocardium Infarction a MiHeart/AMI Study. Trials 9, 41 (2008)].

The main approaches followed for the treatment of ischemic heart disease include those based on the use of myoblasts [Herreros J et al., 2003. Autologous intramyocardial injection of cultured skeletal muscle-derived stem cells in patients with non-acute myocardial infarction. Eur Heart J. 2003 Nov; 24(22):2012-20; Mathur A. et al. 2004. Stem cells and repair of the heart. Lancet 2004 Jul 10-16; 364(9429):183-92] or of bone marrow-derived stem cells [Tomita S et al., 2002. Bone marrow stromal cells contract synchronously with cardiomyocytes in a coculture system. Jpn J Thorac Cardiovasc Surg. 2002 Aug; 50(8):321-4; Fernández-Avilés F et al., 2004. Experimental and clinical regenerative capability of human bone marrow cells after myocardial infarction. Circ Res. 2004 Oct 1; 95 (7) :742-8. Epub 2004 Sep 9]. Nevertheless, there are still a series of critical impediments making their clinical application difficult.

Accordingly, the search for new types of adult stem cells restoring cardiac function continues to be an important challenge.

White adipose tissue is one of the most abundant tissues in the human body and is located in different areas of the body. Said white adipose tissue is made up of two cell populations which can be easily separated, mature adipocytes on one hand and the stromal-vascular fraction (SVF) on the other. The latter is heterogeneous and can be divided into two fractions, as in the case of bone marrow, the stromal fraction and the fraction made up of hematopoietic cells. The stromal fraction is made up of fibroblast-like cells which adhere in culture. Said relatively homogeneous cell population has properties that are similar though not identical to those of bone marrow-derived mesenchymal stem cells [Zuk et al., 2001. Multilineage cells from human adipose tissue: implications for cell-based therapies. Tissue Eng. 2001 Apr; 7(2):211-28]. Said cells, generically referred to as adipose tissue-derived stem cells (ADSCs), are cells which can be easily isolated and cultivated for months with a relatively low duplication time and senescence levels. In the case of subcutaneous adipose tissue-derived stem cells, the latter can be differentiated into several cell types including adipocytes, osteoblasts, chondrocytes and even into cardiomyocytes, in response to specific induction factors of each cell lineage. It has also been published that ADSCs can be a potential source of autologous cells for myocardial repair (W02006/127007).

To date, the clinical use of adult stem cells has given rise to rather modest results with respect to cardiac function recovery. However, clinical trials have demonstrated that cell therapy is safe and is the "proof of concept" that the heart has the capability to regenerate after the therapeutic application of stem cells.

Therefore, even though in recent years considerable progress has been made in the knowledge of myocardial regeneration, a population of adult stem cells which can be used safely and effectively in heart regeneration therapies, thus providing a simple and effective method for repairing myocardial tissue damages, has not been found. Currently, the proportion of patients who can benefit from an effective post-infarction therapy is low; therefore it is necessary to improve the effectiveness of existing therapies, as well as the search for alternatives which can restore cardiac damage, especially for those patients who have suffered multiple infarctions.

### Summary of the Invention

The present invention relates to a novel population of cardiac adipose tissue-derived adult stem cells, preferably from the epicardial area of the myocardium which surprisingly has a certain cardiomyogenic predisposition. Specifically, the invention relates to the use of said population of adult stem cells derived from fatty heart tissue in cell therapy protocols in order to contribute to heart repair in pathophysiological situations.

The invention is based on the finding that this novel population of adult stem cells that is located in the fat surrounding the heart (epicardial and/or pericardial adipose tissue) constitutively expresses *in vitro* a series of cardiospecific markers both at the messenger RNA (mRNA) level, and at the protein level, having a certain predisposition towards a cardiomyocyte lineage. Without the intention of being bound to any hypothesis, it is thought that said predisposition is probably due to their location in intimate contact with the myocardial tissue and due to the environment surrounding them.

The inventors have observed that this novel cell population could have a better cardiomyogenic potential in comparison with that of other populations of stem cells already described, therefore this novel population of adult stem cells isolated from fatty heart tissue is a cell-based reagent potentially useful in cardiac tissue regeneration and in the treatment of situations in which there is a loss of functional myocardial tissue, for example, in patients who have suffered one or more myocardial infarctions or in patients who have developed congestive heart failure.

The inventors have particularly characterized a novel population of human cardiac adipose tissue-derived adult stem cells of the epicardial area by their profile of surface markers and have analyzed the gene (mRNA) and protein expression of different basic components of cardiac muscle cells, which include the cardiac transcription factors GATA-4 and Nkx2.5, the sarcomere components referred to as beta-myosin heavy chain (β-MHC), cardiac troponin I (cTnI), and α-actinin, and the regulators of the electrochemical connection and of the intracellular calcium distribution, connexin-43 (Cx43) and SERCA-2, respectively. The expression of all these markers has been analyzed constitutively, i.e., without the addition to the culture medium of any type of induction factor for the differentiation towards a specific lineage.

A comparative study of the immunophenotypic profile and of the gene and protein expression of cardiospecific markers in substantially homogeneous populations of adult stem cells, from human epicardial adipose tissue (epi-ADSC) and of subcutaneous adipose tissue (sub-ADSC) samples, has been conducted. The isolation and expansion of said cell populations was performed following previously established protocols for subcutaneous fat-derived stem cells. No significant differences were observed with respect to the expression of surface antigens between both cell populations; however, in the comparative assay for the characterization of the gene expression in baseline conditions (Example 3) is clearly showed that said population of epi-ADSC cells surprisingly presented a statistically significant increase of the gene (mRNA) expression levels for the cardiac transcription factor GATA-4 and the Cx43 protein, which is responsible for the electrochemical coupling between adjacent cardiomyocytes with respect to the expression thereof in subcutaneous adipose tissue-derived stem cells (sub-ADSC).

The results obtained in the gene expression analysis were completed with the study of the expression of said cardiospecific markers at the protein level by means of Western Blot and immunofluorescence (Example 4). The results of the immunofluorescence assay show the expression of nuclear GATA-4, β-MHC, SERCA-2, Cx43 and α-actinin, as well as the distribution thereof at the cell level in a population of cardiac adipose tissue-derived stem cells (epi-ADSC). The results of the Western Blot show a comparison of the expression profile of the subcutaneous adipose tissue-derived stem cells (sub-ADSC) with respect to the epi-ADSC cells, confirming a differential expression of the nuclear protein GATA-4 and of Cx43 and how the latter is maintained or increased over cultivation time. A differential expression of the beta-myosin heavy chain (β-MycHC) with the cultivation time is furthermore observed.

The results of the comparative study of the expression of specific genes of cardiomyocyte lineage in the populations of epi-ADSC and sub-ADSC cells demonstrate that the population of epicardial fat-derived stem cells (epi-ADSC) is more committed towards cardiac lineage than the population of subcutaneous fat-derived stem cells from the same individual.

Additional studies conducted by the inventors with said population of human cardiac adipose tissue-derived adult stem cells (cardiac ADSCs) based on the analysis of the initial expression and *in vitro* differentiation experiments have shown that said cardiac ADSCs have an inherent cardiac-type phenotype and cannot differentiate into adipocytes, although they differentiate into an endothelial lineage in culture. Said cardiac ADSCs secrete proangiogenic factors and, when these cells were transplanted into damaged myocardium in myocardial infarction models in rats, the injected cells expressed cardiac and endothelial proteins, increased vascularization, reduced the size of the infarction and accordingly improved *in vivo* cardiac function (Examples 6 and 7); therefore said cardiac ADSCs can be considered as valid candidates for myocardial cell therapy.

Therefore, in one aspect the invention relates to an isolated novel adult stem cell derived from fatty heart tissue of a mammal, constitutively expressing GATA-4 and/or Cx43. In a particular embodiment, said isolated adult stem cell derived from fatty heart tissue of a mammal expresses GATA-4 and/or Cx43 in a constitutive and stable manner during its *in vitro* expansion.

In another aspect the invention relates to an isolated population of said adult stem cells derived from fatty heart tissue of a mammal.

In another aspect the invention relates to a process for obtaining a composition comprising adult stem cells derived from fatty heart tissue of a mammal, constitutively expressing GATA-4 and/or Cx43. The composition obtainable according to said process is an additional aspect of this invention.

In another aspect the invention relates to a method for obtaining differentiated cells from said adult stem cells derived from fatty heart tissue of a mammal, comprising cultivating said stem cells in a suitable specific differentiation medium. The differentiated cells obtainable according to said method are an additional aspect of the invention.

In another aspect the invention relates to a pharmaceutical composition comprising said isolated population of adult stem cells derived from fatty heart tissue or said composition comprising said stem cells derived from fatty heart tissue of a mammal or a composition comprising said differentiated cells obtainable from said adult stem cells derived from fatty heart tissue of a mammal, and a pharmaceutically acceptable vehicle.

In another aspect the invention relates to a biomaterial comprising said isolated population of adult stem cells derived from fatty heart tissue, said composition comprising said stem cells derived from fatty heart tissue of a mammal, said composition comprising said differentiated cells obtainable from said adult stem cells derived from fatty heart tissue of a mammal, or said pharmaceutical composition.

In another aspect the invention relates to the use of said isolated population of adult stem cells derived from fatty heart tissue, or of said composition comprising said stem cells derived from fatty heart tissue of a mammal, or of said composition comprising said differentiated cells obtainable from said adult stem cells derived from fatty heart tissue of a mammal, in the preparation of a pharmaceutical composition for cardiac tissue regeneration, or in the preparation of a pharmaceutical composition for the treatment of an ischemic heart disease, or in the preparation of a pharmaceutical composition for the post-myocardial infarction treatment, or for the treatment of congestive heart failure, or in the preparation of a pharmaceutical composition to stimulate angiogenesis.

In another aspect the invention relates to said isolated population of adult stem cells derived from fatty heart tissue, or of said composition comprising said stem cells derived from fatty heart tissue of a mammal, or of said composition comprising said differentiated cells obtainable from said adult stem cells derived from fatty heart tissue of a mammal, for cardiac tissue regeneration, or for the treatment of an ischemic heart disease, or for the post-myocardial infarction treatment, or for the treatment of congestive heart failure, or to stimulate angiogenesis.

In another aspect the invention relates to a method for cardiac tissue regeneration, or for the treatment of an ischemic heart disease, or for the post-myocardial infarction treatment, or for the treatment of congestive heart failure, or to stimulate angiogenesis, comprising the administration to a subject in need thereof of a therapeutically effective amount of adult stem cells derived from fatty heart tissue, or of said composition comprising said stem cells derived from fatty heart tissue of a mammal, or of said composition comprising said differentiated cells obtainable from said adult stem cells derived from fatty heart tissue of a mammal, or of said pharmaceutical composition.

In another aspect the invention relates to a kit comprising said isolated population of adult stem cells derived from fatty heart tissue or said composition comprising said stem cells derived from fatty heart tissue of a mammal or said composition comprising said differentiated cells obtainable from said adult stem cells derived from fatty heart tissue of a mammal.

In another aspect the invention relates to a method for evaluating *in vitro* the cell response to a biological or pharmacological agent, comprising contacting said agent with an isolated population of adult stem cells derived from fatty heart tissue, or said composition comprising said stem cells derived from fatty heart tissue of a mammal, optionally differentiated into a specific cell type, and evaluating the effects of said agents on said cell population in culture.

In another aspect the invention relates to a process for obtaining growth factors and/or cytokines comprising cultivating said adult stem cells derived from fatty heart tissue of a mammal, or said cells differentiated from said stem cells, under conditions suitable for the expression and production of said growth factors and/or cytokines, and, if desired, separating said growth factors and/or cytokines.

### Brief Description of the Figures

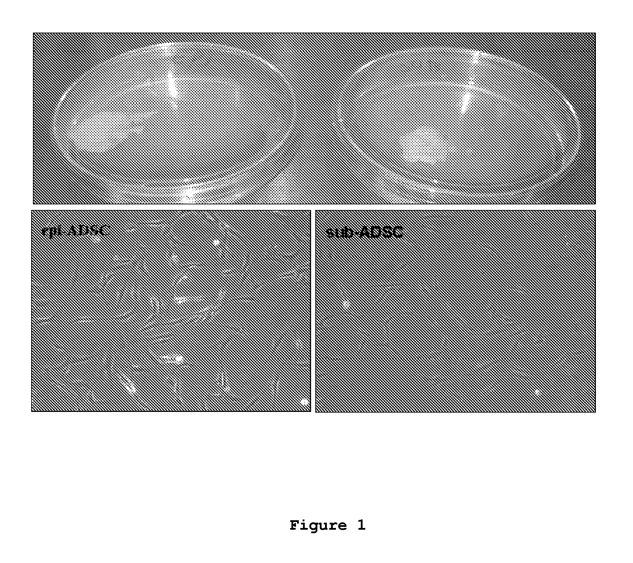

Figure 1 (A) shows a photograph of the extracted fractions of epicardial and subcutaneous fat; Figure 1 (B) shows a microscopic image in which the morphology of the human epicardial adipose tissue-derived adult stem cells (epi-ADSC) can be observed; and Figure 1 (C) shows a microscopic image in which the morphology of the human subcutaneous adipose tissue-derived adult stem cells (sub-ADSC) can be observed.

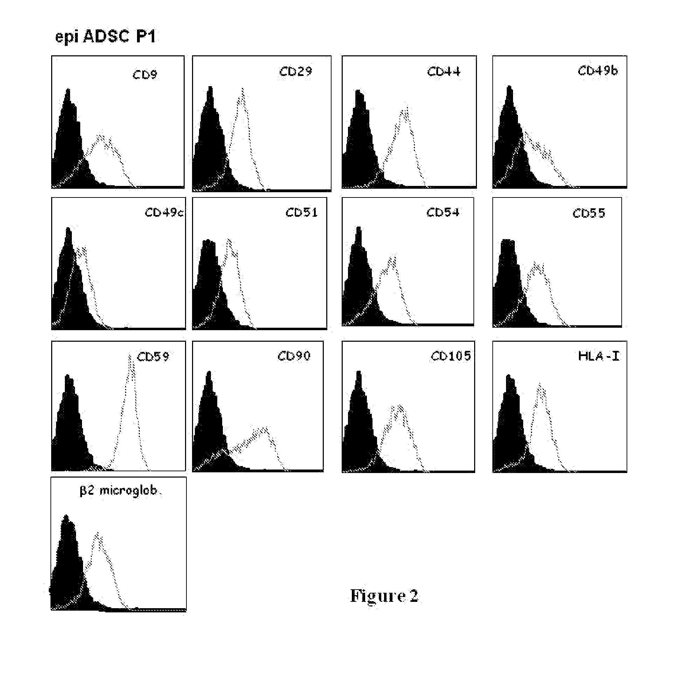

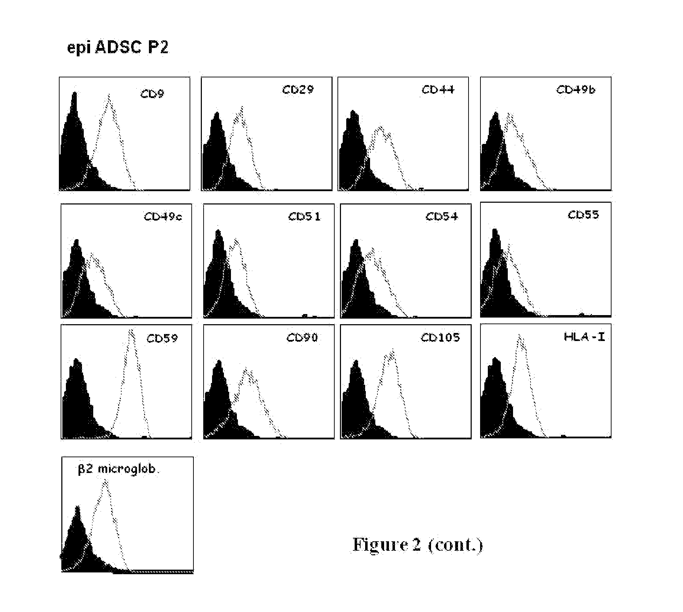

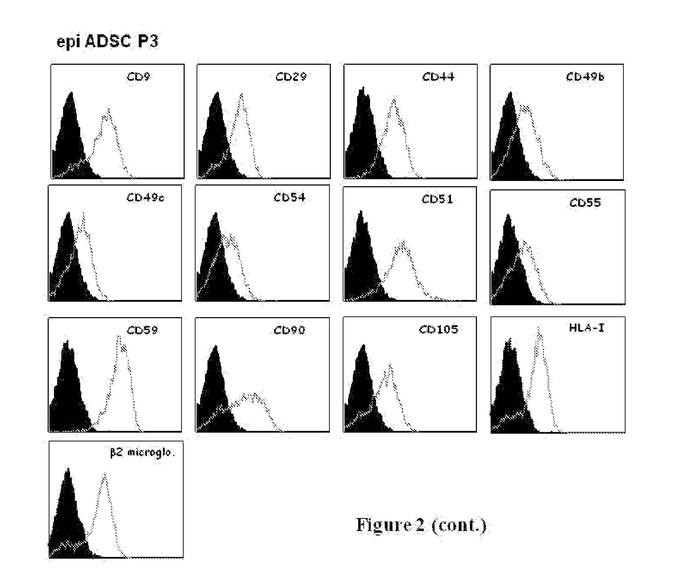

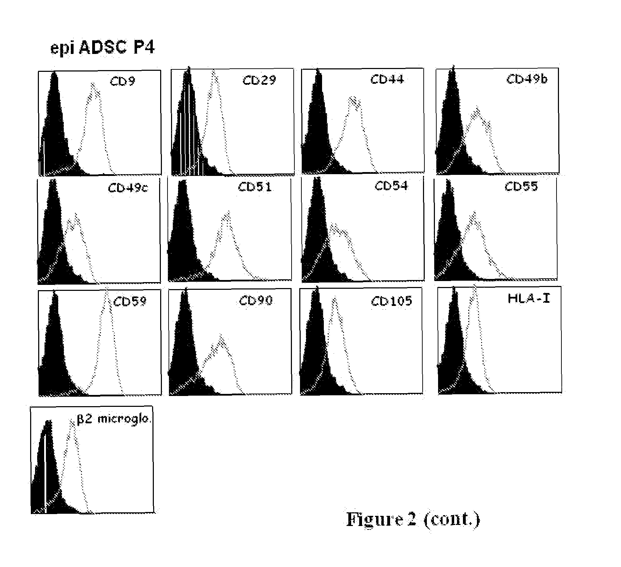

Figure 2 shows the immunophenotypic characterization of human epicardial adipose tissue-derived adult stem cells (epi-ADSC) by means of flow cytometry (FACS). The histograms corresponding to the four patients (P1-P4) under study are depicted [Figure 2A epi-ADSC P1, Figure 2B epi-ADSC P2, Figure2C epi-ADSC P3 and Figure 2D epi-ADSC P4]. The histograms show on the y-axis the fluorescence intensity of the marker under study and on the x-axis the number of cells. Each histogram shows superimposed expression graphs of the control marker (black) and of the marker under study (grey). The degree of lateral displacement of the marker under study with respect to the control on the x-axis indicates how positive the cell sample is for the marker in question.

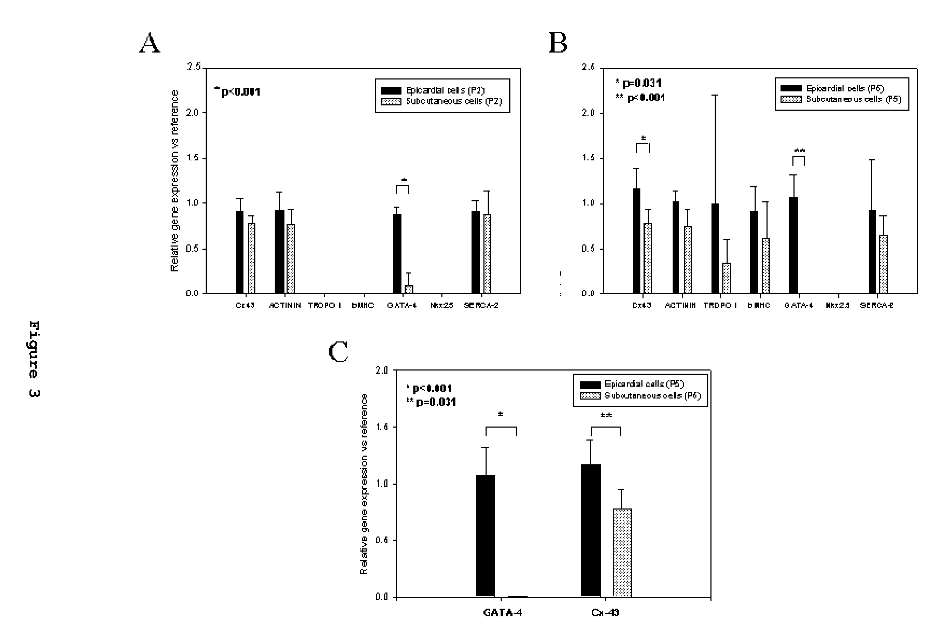

Figure 3 shows the comparative analysis of the constitutive gene expression of cardiospecific markers between the populations of adult stem epi-ADSC and sub-ADSC cells by means of real-time RT-PCR. A differential expression of transcription factor GATA4 (at passage 2 and at passage 5) is observed (Figures 3A, 3B and 3C), increased Cx43 transcript levels (its expression is probably induced by GATA4) in the epi-ADSC stem cells with respect to the sub-ADSC stem cells (Figures 3B and 3C) also being observed at passage 5. For the remaining analyzed cardiac genes (α-actinin, β-MHC, cardiac troponin I, SERCA-2 and Nkx2.5) significant differences were not observed with respect to their expression throughout the cultivation of the cells.

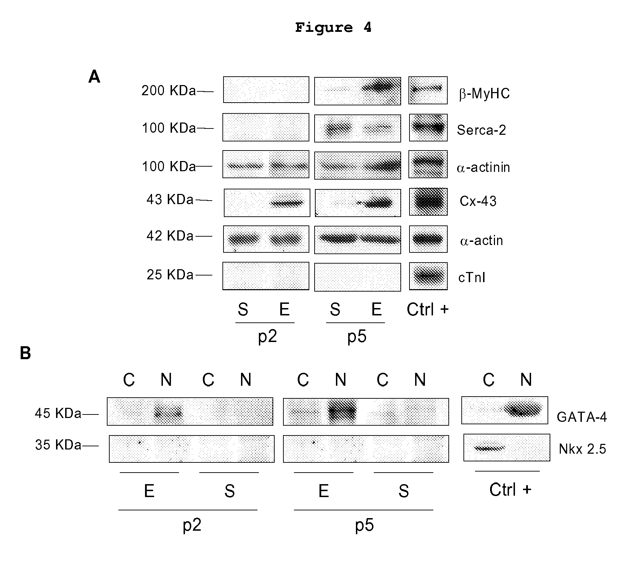

Figure 4 shows the comparative analysis of the expression of cardiospecific markers between the populations of epi-ADSC (E) and sub-ADSC (S) adult stem cells by means of Western Blot. Figure 4A shows the comparative analysis in total extracts. Figure 4B shows the comparative analysis performed in the cytoplasmic (C) and nuclear (N) fractions. An increase of the protein expression levels of Cx43 and GATA-4 by the epi-ADSC stem cells with respect to the sub-ADSC stem cells, both at passage 2 (p2) and at passage 5 (p5) can be observed. A differential expression of β-MHC at passage 5 in the epi-ADSC stem cells is also observed.

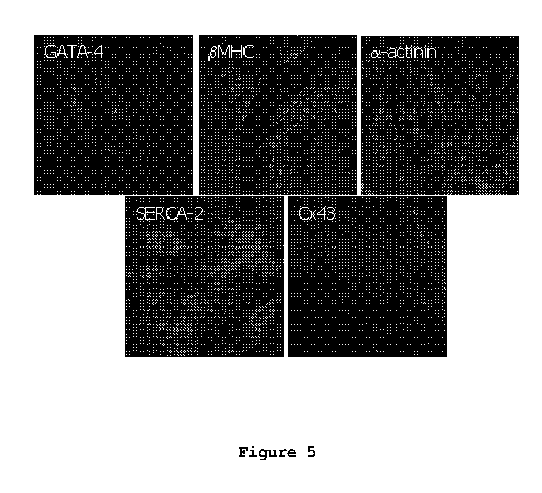

Figure 5 illustrates the expression of cardiospecific markers in the population of epi-ADSC adult stem cells by means of immunofluorescence. The photomicrographs show the detection by means of specific antibodies of the expression of cardiac proteins: GATA-4, β-MHC (aMHC), α-actinin, SERCA-2 and connexin-43 (Cx43). It can be observed that GATA-4 is mostly found in the nucleus of the cells, that β-MHC is structurally well organized forming myofibrils, and that α-actinin, SERCA-2 and Cx43 are diffusely distributed throughout the cells.

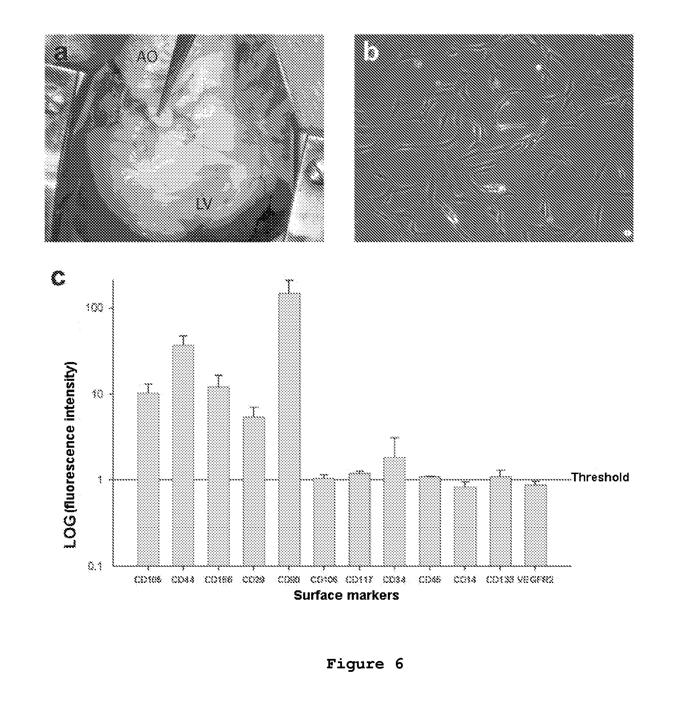

Figure 6 shows the isolation and characterization of cardiac ADSCs. Figure 6a shows a view of a human heart during open-heart surgery; clamps holding the biopsy of cardiac adipose tissue taken next to the proximal right coronary artery are observed. (AO, Aorta; LV, left ventricle). Figure 6b shows a primary culture of cardiac ADSCs before confluence (20x magnification). Figure 6c is a bar graph showing the results of an analysis with flow cytometric immunophenotyping of cardiac ADSCs.

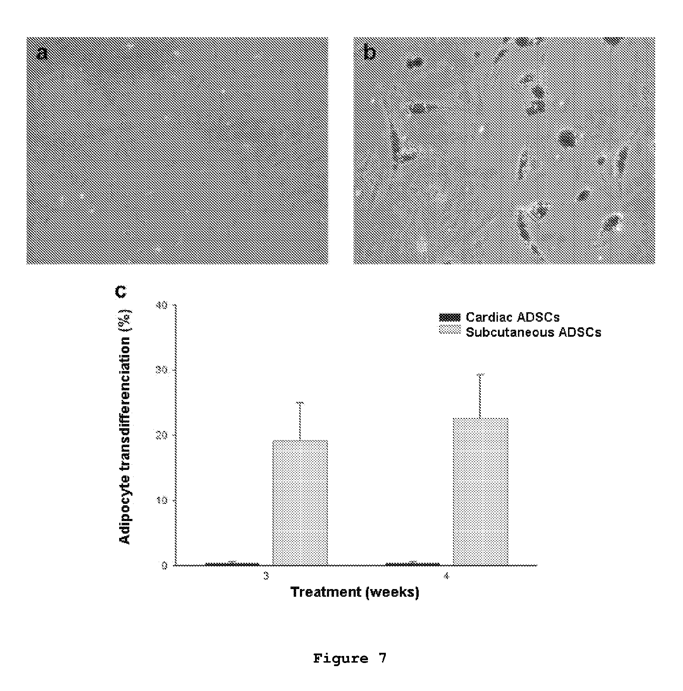

Figure 7 shows the result of an adipogenic differentiation analysis by means of alizarin red S staining of cardiac ADSCs (Figure 7a) and subcutaneous ADSCs (Figure 7b) after the cultivation in medium of adipogenic differentiation. Figure 7c is a bar graph showing the percentage of adipocyte type positive cells after 3 and 4 weeks of treatment for the different cell populations assayed (cardiac and subcutaneous ADSCs).

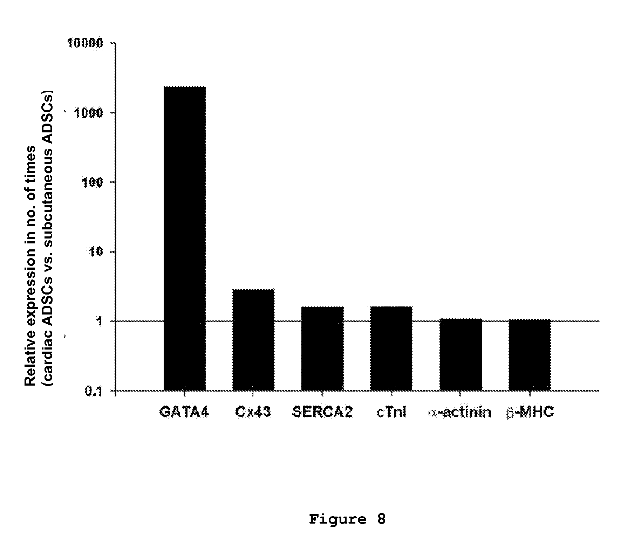

Figure 8 is a bar graph showing the results of the real-time RT-PCR of cardiomyogenic genes in cardiac ADSCs compared to subcutaneous ADSCs. The values indicate the number of times one cell type is expressed with respect to the other cell type from the same patient (in this case, cardiac ADSCs compared to subcutaneous ADSCs). A statistically significant increase of GATA4 and Cx43 transcript is observed in the cardiac ADSCs in comparison with the subcutaneous ADSCs (GATA4, p<0.001; Cx43, p=0.031).

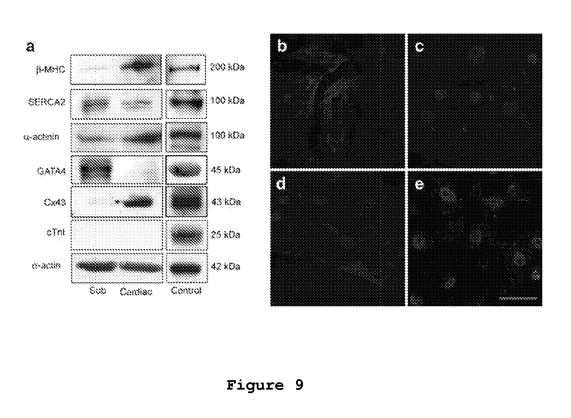

Figure 9 shows the basal expression of cardiac markers in cardiac ADSCs subjected to an assay by means of Western blot and immunocytofluorescence. Figure 9a shows the result of the Western blot analysis of cardiac and subcutaneous (Sub) ADSCs lysates; extracts of adult human heart proteins were used as controls. Figures 9b-9d show the expression of β-MHC, SERCA2 and sarcomeric α-actinin respectively in cardiac ADSCs (red). Figure 9e shows the expression of GATA4 (green) and Cx43 (red) in cardiac ADSCs. The nuclei were stained with Hoescht 33342 (blue) in the panels of Figures 9c and 9d. Scale bar of 50 µm.

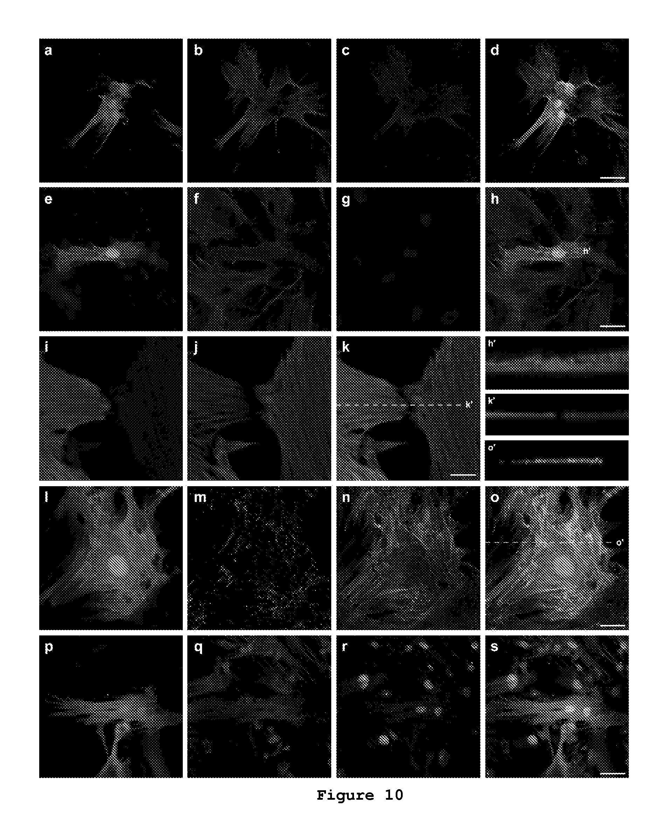

Figure 10 shows the coculture of cardiac ADSCs with neonatal cardiomyocytes. Cardiac eGFP+-ADSCs (green: a, e, i, l and p); cTnI (red: b, f and j); β-MHC (blue: c); nuclear counterstaining with DAPI (cyan: d) (blue: g, h' and s). (h', k' and o') seen in xz mode of dotted lines in h, k and o, respectively. The images were taken with a confocal microscope, showing the co-location of the cardiac markers in the cardiac ADSCs. Cx43 (red: m), sarcomeric α-actinin (cyan: n), SERCA2 (red: q) and GATA4 (cyan: r). (d, h, h', k, k', o, o' and s) are fused images. Scale bar of 50 µm (a-d and p-s), 25 µm (and-h and 1-o) and 10 µm (i-k).

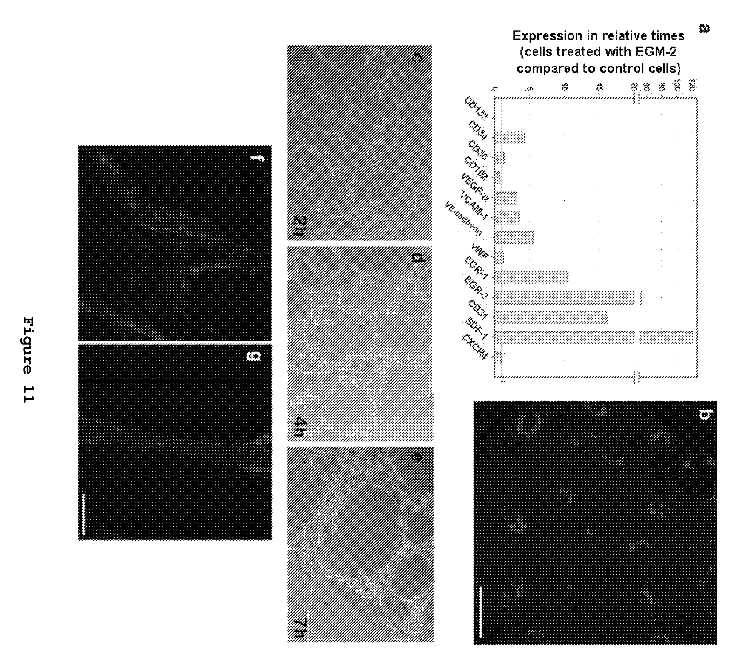

Figure 11 shows that the cardiac ADSCs differentiate into endothelial cells in culture. Figure 11a is a bar graph showing the number of times the proangiogenic markers of cells treated with EGM-2 are expressed compared to untreated control cells, determined by means of real-time RT-PCR. Figure 11b shows the incorporation of DiI-Ac-LDL by the cardiac ADSCs after the differentiation treatment. Figures 11c-11e show the formation of tubules at 2, 4 and 7 hours of cultivation with Matrigel coating of cardiac ADSCs (10x magnification). Figures 11f and 11g show the GSLI isolectin B4 staining of the tubules formed in the coating with Matrigel. Scale bar of 50 µm.

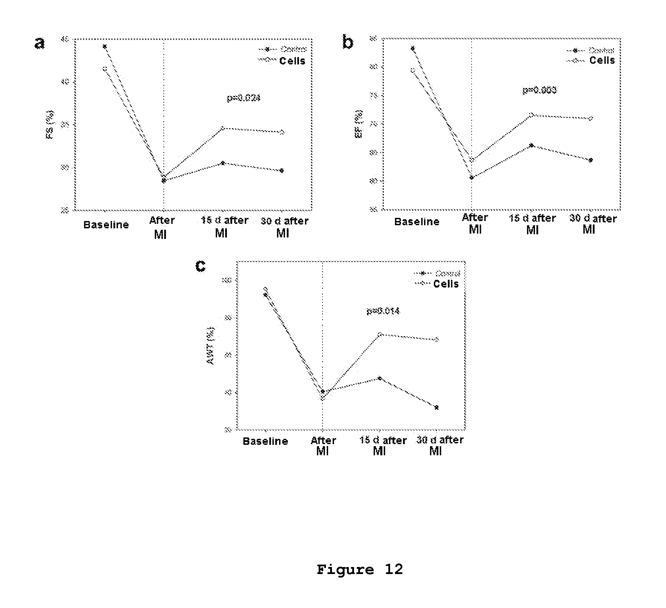

Figure 12 shows the values of different echocardiographic functional parameters: fractional shortening (FS) [Figure 12a]; ejection fraction (EF) [Figure 12b]; and anterior wall thickness (AWT) [Figure 12c].

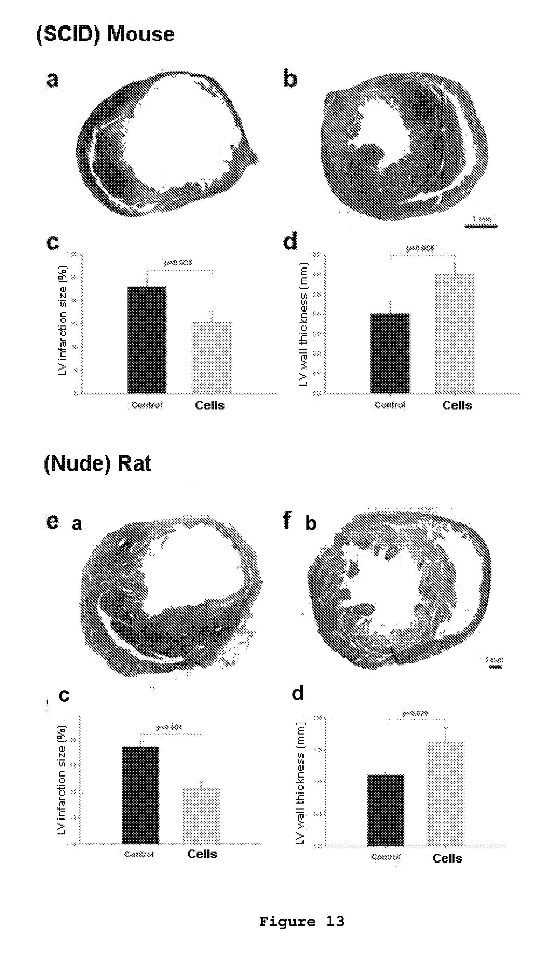

Figure 13 shows the result of the morphometric analysis of rat hearts. Figures 13a and 13b show the results of Masson's trichrome staining of cross sections of rat hearts through infarcted myocardium 30 days after surgery (e, control; f, treated). Figures 13c and 13d show the infarction size percentage of the LV of rats and the LV wall thickness in the control groups and in groups treated with cells.

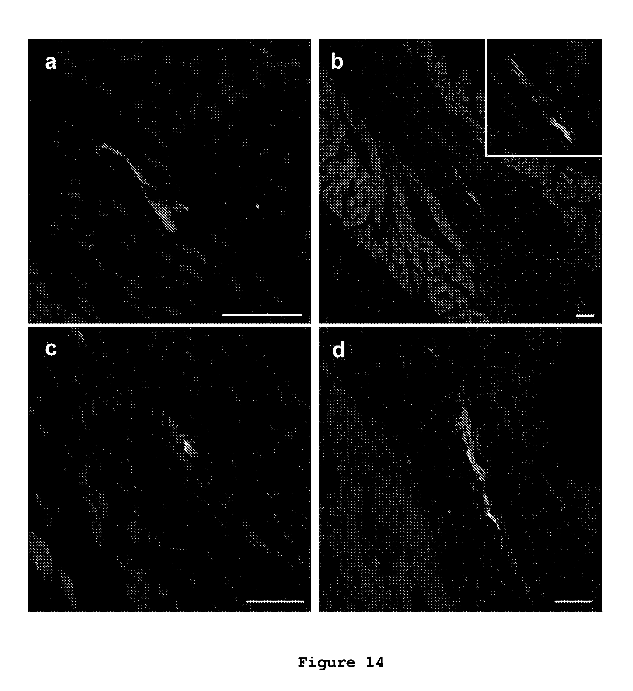

Figure 14 shows the results of the grafting of human cardiac ADSCs in the hearts of rats with infarction, in which a cardiac and endothelial *in vivo* differentiation can be observed. Figure 14a shows the cardiac eGFP+-ADSCs injected in the myocardium in green and the result of the immunostaining for human nuclear antigen (HNA, red) is shown to confirm the human origin of the injected cardiac eGFP+-ADSCs. Figures 14b-14d show the result of the immunostaining in sections of a heart in which cardiac ADSCs were injected for cTnI (b), sarcomeric α-actinin (c) and CD31 (d) (all in red). It is important to observe the distribution of the cells throughout the ischemic tissue despite the injection in the border area. Scale bars of 50 µm.

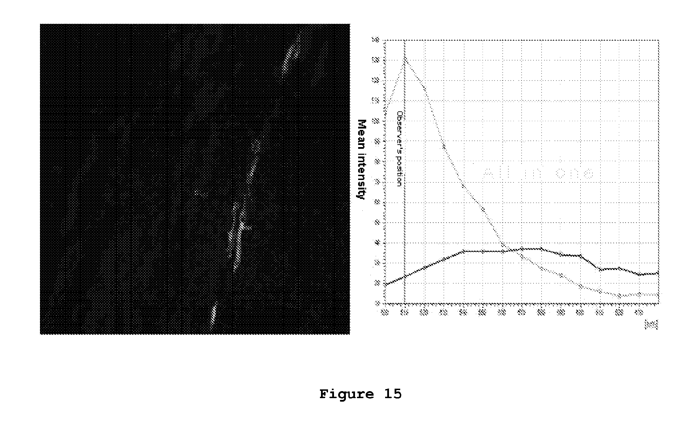

Figure 15 shows the result of the measurement of the emission spectrum of eGFP+ (ROI1) and background (ROI2) cells.

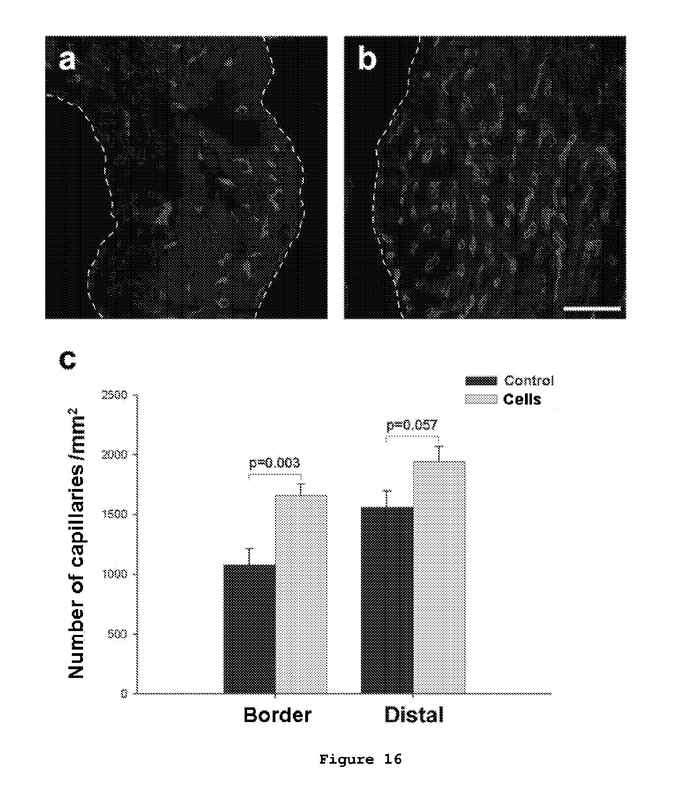

Figure 16 shows the results of the capillary density analysis in infarcted myocardium, specifically capillaries in the border area of control myocardia (Figure 16a) and in myocardia treated with cardiac ADSCs (Figure 16b) stained with GSLI isolectin B4. Figure 16c is a bar graph showing the number of capillaries per mm² in the control group and in the group treated with cardiac ADSCs in the border and distal areas of the infarction. Significant differences were not observed in the distal area. Scale bar of 50 µm.

### Detailed Description of the Invention

The present invention generally relates to a substantially homogenous population of cardiac adipose tissue-derived adult stem cells, present in the epicardial and/or pericardial area. The inventors have observed that said cell population has a certain predisposition to cardiac lineage, having greater cardiomyogenic potential in comparison with other stem cells of a different origin. Said cell population is therefore potentially useful in the cardiac tissue regeneration. Specifically, the invention relates to the use of said population of adult stem cells derived from fatty heart tissue in cell therapy protocols which contribute to heart repair in pathophysiological situations. Said cell population can be used in the preparation of a pharmaceutical composition or in a biomaterial for myocardial regeneration in those situations in which there is a loss of the contractile capability of the myocardium, for example, in the treatment of patients who have suffered a myocardial infarction and/or have developed congestive heart failure.

### Definitions

For the purpose of aiding in the understanding of the present description, the meaning of some terms and expressions as they are used in the context of the invention will be explained below. Additional definitions will be included together with the description when necessary.

The term "subject" relates to an animal, preferably a mammal, including non-primates (e.g., cows, pigs, horses, cats, dogs, rats or mice) and primates (e.g., monkeys, human beings). In a preferred embodiment, the subject is a human being.

The term "adult stem cells" relates to cells present in a differentiated tissue having characteristics of stem cells. An adult stem cell is a non-differentiated cell found in a differentiated tissue and has the capability for proliferation and differentiation into one or more cell types. The adult stem cells are present in different adult tissues, their presence being widely described in bone marrow, adipose tissue, blood, cornea, retina, brain, skeletal muscle, dental pulp, gastrointestinal epithelium, liver and skin.

The term "adipose tissue" or "fatty tissue", used indistinctly in this description, relates to the mesenchymal tissue formed by the association of cells accumulating lipid in their cytoplasm: adipocytes. On one hand, adipose tissue performs mechanical functions, including serving as a buffer, protecting and keeping in place internal organs, as well as other more external structures of the body, and on the other hand it also performs metabolic functions. Two types of adipose tissue can be distinguished in mammals: brown adipose tissue or brown fat and white adipose tissue. These tissues are recognized as different tissues in metabolic terms and in terms of cellular composition. In newborns and infants under the age of 6 months, brown fat is involved in thermogenesis as a compensatory mechanism during exposure to a cold environment whereas in adults, said thermogenesis is mediated by thyroid activity. An article has recently been published in which neonatal brown fat as a source of progenitor (stem) cells of cardiomyocytes and their potential use in regenerative cardiac therapy is identified (Yamada et al., 2006. Cardiac progenitor cells in brown adipose tissue repaired damaged myocardium. Biochem Biophys Res Commun. 2006 Apr 7; 342(2):662-70. Epub 2006 Feb 10). The set of studies performed on white adipose tissue shows that it is directly or indirectly involved in all the main functions of the organism. It is a very heterogeneous tissue consisting of a number of cell populations interacting with one another. In this context it is necessary to consider the difference in the biology and plasticity of the cells depending on their location. The comparison of masculine and feminine obesity shows that the development of adipose tissue, depending on its location, has different effects on the organism. The differentiation potential of the cells also depends on the location thereof. In a comparative study between the differentiation potential of the stromal cell population of adipose tissue depending on the origin of the deposit, it is demonstrated that in the case of mice, the adipose tissue having the greatest capability for differentiation is subcutaneous adipose tissue (Prunet-Marcassus et al., 2006. From heterogeneity to plasticity in adipose tissues: site-specific differences. Exp Cell Res. 2006 Apr 1; 312(6):727-36. Epub 2005 Dec 28).

The term "fatty heart tissue" relates to cardiac adipose tissue which, due to its anatomical location, can be distinguished into epicardial adipose tissue (covering the myocardium) or pericardial adipose tissue (in the pericardial area, i.e., in the vicinity of the heart). It is known that adipose tissue is a highly complex endocrine organ which generates molecules with both local and systemic effects. Despite the similarity of their qualitative properties, it is currently known that different types of adipose tissue, particularly subcutaneous and visceral adipose tissue deposits have a profile of differential expression of certain proteins, suggesting differential characteristics in the production of active biological molecules (Dusserre E. et al.; 2000. Differences in mRNA expression of the proteins secreted by the adipocytes in human subcutaneous and visceral adipose tissues. Biochim Biophys Acta. 2000 Jan 3; 1500(1):88-96).

The term "constitutive expression" or "basal expression" relates to the expression of a gene in the absence of factors inducing the expression thereof. Constitutive expression genes are understood as those genes which are transcribed continuously.

When the term "isolated" relates to a cell population, it relates to a cell population isolated from an organ or tissue of an animal, including human beings, which is substantially free of other cell populations generally associated with said cell population *in vitro* or *in vivo.* Generally, a cell population "substantially free" of others is obtained when it is separated from at least 50%, preferably from at least 60%, more preferably from at least 70%, even more preferably from at least 80%, still more preferably from at least 90%, and, still even more preferably from at least 95%, 96%, 97%, 98% or even 99% of other cell populations generally associated with said cell population *in vitro* or *in vivo.*

The term "ischemic heart disease" relates to the disease resulting from the inability of the coronary arteries to carry the necessary oxygen to a determined territory of the heart muscle, making the working of said muscle difficult. The most frequent causes of the alteration of the coronary arteries are arteriosclerosis or atherosclerosis. These two situations make it difficult for the blood to reach the cells of the heart, which are very sensitive to the reduction of the blood supply. Coronary heart disease or ischemic heart disease manifests when the amount of oxygen reaching the heart is insufficient. Its main consequences are myocardial infarction, angina pectoris, coronary insufficiency, myocardial ischemia and sudden death.

The term "heart failure", "congestive heart failure" or "CHF" relates to a chronic disease in which the heart cannot pump enough oxygenated blood to meet the needs of other organs of the body. As a result, the vital organs of the organism do not receive enough oxygen and nutrients, and the wastes from the organism are eliminated more slowly. In the long run, the vital systems stop working. Heart failure is generally a symptom of an underlying heart problem.

The term "myocardial infarction" or "acute myocardial infarction" or "AMI" relates to an ischemic necrosis of part of the myocardium due to the obstruction of one or several coronary arteries or their branches. Myocardial infarction is characterized by the loss of functional cardiomyocytes, the myocardial tissue being irreversibly damaged. The myocardium, or heart muscle, suffers an infarction when advanced coronary disease exists, in a particular case this occurs when an atheromatous plaque located inside a coronary artery ulcerates or ruptures, causing an acute obstruction of that vessel.

The term "cardiac regeneration", "cardiac tissue regeneration" or "myocardial regeneration" relates to the repair of the loss of cardiac tissue cell mass by means of the implantation of stem cells capable of proliferating and differentiating into cardiomyocytes, regenerating the damaged myocardial tissue and cardiac function.

As used in this document, the terms "treat, "treatment" and "treating" relate to the improvement, cure or remedy of the pathophysiological situation, which results from the administration of the pharmaceutical composition provided by the present invention, comprising said population of adult stem cells derived from fatty heart tissue, to a subject in need of said treatment.

### Stem cells of the invention

The present invention is based on the identification of a novel population of cardiac adipose tissue-derived adult stem cells having a certain cardiomyogenic predisposition, therefore they can be used in cell therapy protocols, particularly in cell therapy protocols in order to contribute to the repair and/or regeneration of myocardial tissue in pathophysiological situations in which there has been a loss of functional cardiac tissue.

Therefore, in one aspect the invention relates to an isolated adult stem cell derived from fatty heart tissue of a mammal, hereinafter stem cell of the invention, constitutively expressing GATA-4 and/or connexin 43 (Cx43).

The stem cells of the invention, occasionally also identified in this description as "cardiac ADSCs", come from a source of cardiac adipose tissue, e.g., epicardial or pericardial adipose tissue, such as the stromal fraction of said cardiac adipose tissue of a mammal, such as a rodent, a primate, etc., preferably of a human being. In a particular embodiment, the stem cells of the invention are obtained from human epicardial adipose tissue.

The stem cells of the invention can be autologous, allogeneic or xenogeneic cells. In a particular and preferred embodiment, said cells are autologous and are isolated from the cardiac adipose tissue of the subject to whom they will be administered.

In addition to by their origin, the stem cells of the invention are characterized by constitutively expressing GATA-4 and/or Cx43. In a particular embodiment, the stem cells of the invention constitutively express GATA-4 and Cx43.

GATA-4 is a transcription factor member of the GATA family of zinc finger transcription factors. The members of this family recognize the GATA motif, which is present in the promoters of several genes. It is thought that said protein participates in the regulation of genes involved in embryogenesis, as well as in cardiac differentiation and function. Mutations in the gene encoding said GATA-4 protein have been associated with defects in the heart septum.

Connexin 43 (Cx43), also referred to as GJA1 (gap junction protein), is a member of the connexin family. Said protein is a component of the intercellular gap junctions, which are made up of a group of intercellular channels providing a pathway for the diffusion of low-molecular weight material from one cell to another. The Cx43 protein is one of the main proteins in the gap junctions of the heart which is thought to play a crucial role in the synchronization of the contraction of the heart, as well as in embryonic development.

As previously discussed, the skeletal muscle is considered to be a potential source for obtaining cells for myocardial regeneration; however, the skeletal muscle does not express the Cx43 protein and the gap junctions existing between cardiomyocytes are not formed. Therefore, the contraction between the resulting myotubes and the adjacent myocardium is asynchronous. This lack of electric coupling is what possible explains the onset of malignant arrhythmias in some clinical series (Herreros J et al., 2003. Autologous intramyocardial injection of cultured skeletal muscle-derived stem cells in patients with non-acute myocardial infarction. Eur Heart J. 2003 Nov; 24(22):2012-20).

In a particular and preferred embodiment, the constitutive expression of GATA-4 and/or Cx43 in said stem cell of the invention is maintained stable during its *in vitro* expansion.

As used herein, the "stable" expression of a gene or a protein by a cell "during its *in vitro* expansion" relates to the expression of a gene or of its expression product (protein) by a cell being maintained at substantially the same level during at least two passages of cell culture *in vitro*; i.e., the cell is cultivated *in vitro* under suitable conditions (culture medium, temperature, atmosphere, dilution, culture medium change, etc.) for its *in vitro* expansion, such as cultivation in α-MEM culture medium supplemented with 10% FBS, 1 mM L-glutamine and 1% penicillin-streptomycin at 37°C under air atmosphere with 5% CO₂, until pre-confluence, replacing the culture medium every 3 or 4 days, as mentioned in Example 1.

The stem cells of the invention can be autologous, allogeneic or xenogeneic cells. In a particular and preferred embodiment, said cells are autologous and are isolated from the cardiac adipose tissue of the subject to whom they will be administered.

In a particular embodiment, said stem cell of the invention constitutively expresses beta-myosin heavy chain (β-MHC), constitutively absent in other populations of stem cells already described as well as in subcutaneous adipose tissue-derived stem cells (sub-ADSCs) obtained from the same subject as the adult stem cells derived from fatty heart tissue (stem cells of the invention). It is known that this protein plays an essential role in the contraction of the heart muscle due to the fact that it forms part of the sarcomere (contractile apparatus of the cardiomyocytes).

In another particular embodiment, the stem cell of the invention constitutively expresses the SERCA-2 protein throughout its cultivation *in vitro.* This sarcoplasmic protein (SERCA-2) is a Ca²⁺-ATPase pump and is responsible for the regulation of the intracellular distribution of calcium inside the cardiac muscle cell. This function is also basic for the correct contraction of the heart muscle.

Furthermore, the stem cell of the invention can also be characterized by the expression or non-expression of a series of surface markers, such as CD14, CD29, CD34, CD44, CD59, CD90, CD105, CD106 and CD117, and optionally of the markers CD45, CD133, CD166 and VEGFR2.

In a particular embodiment, the stem cell of the invention is **characterized in that** it furthermore expresses one or more surface markers selected from CD29, CD44, CD59, CD90 and CD105; i.e., the stem cell of the invention is positive for at least one, two, three, four, or preferably all the surface markers CD29, CD44, CD59, CD90 and CD105. In another particular embodiment, the stem cell of the invention is **characterized in that** it furthermore expresses the surface marker CD166. Therefore, in another particular embodiment, the stem cell of the invention is **characterized in that** it furthermore expresses one or more surface markers selected from CD29, CD44, CD59, CD90, CD105 and CD166; i.e., the stem cell of the invention is positive for at least one, two, three, four, five, or preferably all the surface markers CD29, CD44, CD59, CD90, CD105 and CD166.

In another particular embodiment, the stem cell of the invention is **characterized in that** it does not express a surface marker selected from CD14, CD34, CD106, CD117 and combinations thereof; i.e., the stem cell of the invention is negative for at least one, two, three, or preferably all the surface markers CD14, CD34, CD106 and CD117. In another particular embodiment, the stem cell of the invention is **characterized in that** it does not express (or it very weakly expresses) the surface marker VEGFR2. Therefore, in another particular embodiment, the stem cell of the invention is **characterized in that** it is negative for at least one, two, three, four, or preferably all the surface markers CD14, CD34, CD106, CD117 and VEGFR2.

In another particular embodiment, the stem cell of the invention is characterized, in addition to by its origin and by the constitutive expression of GATA-4 and/or Cx43, in that (i) it expresses all the surface markers CD29, CD44, CD59, CD90 and CD105, and (ii) it does not express any of the surface markers CD14, CD34, CD106 and CD117.

In another particular embodiment, the stem cell of the invention is characterized, in addition to by its origin and by the constitutive expression of GATA-4 and/or Cx43, in that (i) it expresses all the surface markers CD29, CD44, CD90, CD105 and CD166 and (ii) it does not express any of the surface markers CD14, CD34, CD106, CD117 or VEGFR2.

In another particular embodiment, the stem cell of the invention is characterized, in addition to by its origin and by the constitutive expression of GATA-4 and/or Cx43, in that (i) it expresses all the surface markers CD29, CD44, CD59, CD90, CD105 and CD166, and (ii) it does not express any of the surface markers CD14, CD34, CD106, CD117 or VEGFR2.

In a specific embodiment, the adult stem cell of the invention is an isolated adult stem cell derived from cardiac fatty (adipose) tissue of a mammal, preferably of a human being, which:
a) constitutively expresses GATA-4 and/or Cx43;
b) constitutively expresses β-MHC;
c) expresses all the surface markers CD29, CD44, CD59, CD90 and CD105; and
d) does not express any of the surface markers CD14, CD34, CD106 and CD117.

In another specific embodiment, the adult stem cell of the invention is an isolated adult stem cell derived from cardiac fatty (adipose) tissue of a mammal, preferably of a human being, which:
a) constitutively expresses GATA-4 and/or Cx43;
b) constitutively expresses β-MHC;
c) expresses all the surface markers CD29, CD44, CD90, CD105 and CD166; and
d) does not express any of the surface markers CD14, CD34, CD106, CD117 or VEGFR2.

In another specific embodiment, the adult stem cell of the invention is an isolated adult stem cell derived from cardiac fatty (adipose) tissue of a mammal, preferably of a human being, which:
a) constitutively expresses GATA-4 and/or Cx43;
b) constitutively expresses β-MHC;
c) expresses all the surface markers CD29, CD44, CD59, CD90, CD105 and CD166; and
d) does not express any of the surface markers CD14, CD34, CD106, CD117 or VEGFR2.

In a particular and preferred embodiment, the constitutive expression of GATA-4 and/or Cx43 in said stem cell of the invention is maintained stable during its *in vitro* expansion.

The expression of the genes and proteins of interest (e.g., GATA-4, Cx43, α-MHC, β-actinin, etc.) can be determined by conventional methods known by persons of ordinary skill in the art either at the nucleic acid level (e.g., mRNA level) or at the protein level.

In a particular embodiment, the expression of a given gene can be determined by means of the analysis of its gene expression without adding to the culture medium any component capable of inducing differentiation to a specific lineage, i.e., under constitutive expression conditions. By way of a non-limiting illustration, the expression of said genes in a cell can be analyzed by means of conventional techniques such as real-time RT-PCR, Northern blot or DNA microarrays. Real-time RT-PCR is a variant of reverse transcription-polymerase chain reaction allowing a quantitative detection of the gene as it is being amplified. Real-time RT-PCR is used in Examples 3 and 6 to determine the gene expression levels (mRNA) of the cardiospecific proteins of interest. The Northern blot technique allows the identification, location and quantification of mRNA sequences by means of the transference of all the mRNA from a gel to a nitrocellulose or nylon membrane. The presence of a particular mRNA is detected by hybridization with a suitable probe. The DNA microarray technique is based on the use of a solid surface to which a series of DNA fragments are bound. The superficies used to fix the DNA are quite varied (e.g., glass, plastic and even silicon chips); this technique allows ascertaining the expression of a number of genes, the expression levels of a large number of genes being able to be monitored simultaneously.

Alternatively, the expression of a protein can be analyzed by means of immunological techniques, e.g., ELISA, Western Blot or immunofluorescence. The Western Blot technique is based on the detection of proteins previously separated by electrophoresis and immobilized on a membrane, generally a nitrocellulose membrane, by means of incubation with a specific antibody and a developing system, e.g., chemiluminescence. The analysis by means of immunofluorescence relates to the use of an antibody specific for a protein of interest for the analysis of its expression and subcellular location by means of microscopy. Generally, the cells under study are previously fixed with paraformaldehyde and permeabilized with a non-ionic detergent. Western Blot and immunofluorescence techniques are used in Examples 4 and 6. The ELISA technique is based on the use of antigens or antibodies marked with enzymes such that the resulting conjugates have both immunological and enzymatic activity. Since one of the components is marked and insolubilized on a support, the antigen-antibody reaction is immobilized and, therefore, can be easily developed by means of the addition of a specific substrate producing a reaction that is quantifiable by means of, for example, spectrophotometry. This technique does not allow the exact subcellular location nor the determination of the molecular weight of the proteins studied but it does allow a very specific and highly sensitive detection of proteins of interest for example in biological fluids of clinical interest (serum, cell culture supernatants, ascitic fluid, etc...).

The phenotypic markers of the stem cells of the invention can also be identified by any suitable technique normally based on a positive/negative selection. In a particular embodiment, antibodies, preferably monoclonal antibodies, can be used against said phenotypic markers the presence or absence of which in the stem cells of the invention must be confirmed to additionally characterize the stem cells of the invention by means of their immunocytochemical profile, although other conventional techniques known by persons skilled in the art can also be used. Therefore, in a particular embodiment monoclonal antibodies are used against at least cell surface markers CD29, CD44, CD59, CD90 and CD105, for the purpose of confirming the presence of said markers in the selected cells or the detectable expression levels of at least one of said markers, and preferably of all of them, and monoclonal antibodies are used against at least CD14, CD34, CD106 and CD117, to confirm the absence thereof. In another particular embodiment, monoclonal antibodies are used against at least cell surface markers CD29, CD44, CD90, CD105 and CD166, for the purpose of confirming the presence of said markers in the selected cells or the detectable expression levels of at least one of said markers, and preferably of all of them, and monoclonal antibodies are used against at least CD14, CD34, CD106, CD117 and VEGFR2, to confirm the absence thereof. Likewise, in another particular embodiment, monoclonal antibodies are used against at least CD14, CD29, CD34, CD44, CD59, CD90, CD105, CD106, CD117, CD166 and VEGFR2, for the purpose of confirming the presence or absence of said markers in the selected cells or the detectable expression levels of at least one of said markers, and preferably of all of them.

Said monoclonal antibodies are known or can be obtained by any person skilled in the art by means of conventional processes. A manner of carrying out the immunophenotypic characterization of a population of stem cells provided by this invention is described in Examples 2 and 6 by way of a non-limiting illustration.

If desired, the stem cell of the invention can be genetically modified by any conventional method including, by way of a non-limiting illustration, processes of transgenesis, deletions or insertions in its genome which modify the expression of genes that are important for its basic properties (proliferation, migration, transdifferentiation, etc.). Thus, for example, it is known that the adult stem cells expanded *ex vivo* or transplanted within the damaged tissues age quickly due to the shortening of their telomeres. To prevent this and other phenomena, it can be desirable to transduce the cells using, for example, retroviral viral particles containing gene constructs the expression of which counteracts the effect of this and other unwanted alterations.

The stem cells of the invention have the capability for proliferation and self-renewal therefore they can be expanded in *vitro* (*ex vivo*) once they are isolated and characterized. Therefore, once the stem cells of the invention are isolated, they can be maintained and proliferate *in vitro* in a suitable culture medium. By way of a non-limiting illustration, said medium comprises α-MEM medium (Minimum Essential Medium eagle-alpha modification (Sigma Ref. M4526). Eagle, H. Media for animal cell culture. Tissue Culture Association Manual. 3.517-520.1976), antibiotics and glutamine, and it is complemented with fetal bovine serum (FBS). It will depend on the experience of each person of ordinary skill in the art to modify or modulate the concentrations of the media and/or the media supplements as required for the cells used. The sera often contain cell components and factors which are necessary for cell viability and expansion. Illustrative, non-limiting examples of such sera, include FBS, bovine serum (BS), calf serum (CS), fetal calf serum (FCS), neonatal calf serum (NCS), goat serum (GS), horse serum (HS), pig serum, sheep serum, rabbit serum, rat serum (RS), etc. If the stem cells of the invention are of human origin, supplementing the cell culture medium with a human, preferably autologous, serum is also contemplated. It is understood that the sera can be inactivated by heat if considered necessary to inactivate the cascade components of the supplement. The modulation of serum concentrations, the removal of serum from the culture medium to promote the survival of one or more desired cell types can also be used. In another embodiment, the stem cells of the invention can be expanded in a culture medium with a defined composition, in which the serum is replaced by a combination of serum albumin, transferrin, selenium and recombinant proteins including, though not being limited to, insulin, platelet-derived growth factor and a growth factor, e.g., basic fibroblast growth factor (bFGF).

Many cell culture media already contain amino acids; nevertheless, some require being supplemented before cultivating the cells. Illustrative, non-limiting examples of said amino acids include L-alanine, L-arginine, L-aspartic acid, L-cysteine, L-cystine, L-glutamic acid, L-glutamine, L-glycine, etc. Antimicrobial agents are also normally used in the cultivation of cells to mitigate a possible bacterial, mycoplasma and/or fungal contamination. The antibiotic or antimycotic compounds used are normally mixtures of penicillin and streptomycin, although other antibiotic or antimycotic compounds can also be included, such as, for example, amphotericin, ampicillin, gentamicin, bleomycin, hygromycin, kanamycin, mitomycin, etc. Hormones can also be added to the cell culture, including though not limited to D-aldosterone, diethylstilbestrol (DES), dexamethasone, b-estradiol, hydrocortisone, insulin, prolactin, progesterone, somatostatin/human growth hormone (HGH), etc.

The maintenance of the stem cells of the invention can require the incorporation of cell factors allowing the cells to remain in a non-differentiated form. It will be evident for the person of ordinary skill in the art that said factors inhibiting cell differentiation must be removed from the culture medium before beginning to differentiate the stem cells of the invention into differentiated cells. It is also evident that not all the cells will require these factors. In fact, these factors can cause unwanted effects, depending on the cell type.

If desired, the stem cells of the invention can be clonally expanded using a suitable process for cloning cell populations. By way of illustration, a proliferated population of stem cells of the invention can be physically collected and seeded on a separate plate (or in the wells of a "multi-well" plate). Stem cells of the invention can alternatively be sub-cloned into a "multi-well" plate in a statistical ratio to facilitate the operation of placing a single cell in each well (e.g., from approximately 0.1 to about one cell/well or even about 0.25 to 0.5 cells/well, for example 0.5 cells/well). Of course, the cells can be cloned at a low density (for example, in a Petri dish or other suitable substrate) and be isolated from other cells using devices such as cloning rings. The production of a clonal population can be expanded in any suitable culture medium. In any case, the isolated cells can be cultivated to a suitable point when their developmental phenotype can be evaluated. Any of the steps and processes for isolating the stem cells of the invention can be carried out manually, if desired; alternatively, the suitable devices known by persons of ordinary skill in the art can be used to facilitate the isolation of the cells.

The analysis of the capability of the stem cells of the invention of differentiating into one or more cell lineages or types can be evaluated by means of conventional processes of induction of differentiation known by persons of ordinary skill in the art. To that end, the stem cells are generally subjected to the suitable specific differentiation protocols for each cell type or lineage, including the cultivation of the stem cells in the suitable specific differentiation media.

After analyzing the results obtained in preliminary differentiation studies following the usual protocols for inducing specific differentiation into an adipocyte, osteocyte and chondrocyte, the inventors consider that the stem cells of the invention have a reduced (lower) capability for differentiation with respect to that of other populations of adult mesenchymal stem cells also positive for the markers CD29, CD44, CD59, CD90 and CD105, e.g. subcutaneous adipose tissue-derived stem cells (sub-ADSCs). Specifically (Example 5), the stem cells of the invention were subjected to adipogenic, osteogenic and chondrogenic differentiation protocols previously established for subcutaneous adipose tissue-derived stem cells [Zuk et al., 2002. Human adipose tissue is a source of multipotent stem cells. Mol Biol Cell. 2002 Dec; 13(12):4279-95] and it was observed that the stem cells of the invention had a lower capability for differentiation into said lineages with respect to subcutaneous adipose tissue-derived stem cells. Nevertheless, the possibility of obtaining a greater capability for differentiation by means of modifications in the specific adipogenic, chondrogenic and osteogenic lineage differentiation protocols used cannot be disregarded. Thus, although there is no intention to be linked by any conclusion, there seem to be indicia which lead to think that the stem cells of the invention have a profile of differentiation into mesenchymal lineages different from that of other populations of adult mesenchymal stem cells also positive for the markers CD29, CD44, CD59, CD90 and CD105. In fact, additional assays conducted by the inventors seem to confirm that the stem cells of the invention do not differentiate into adipocytes (Example 6), which indicates lower plasticity and a higher degree of commitment, unlike the subcutaneous adipose tissue-derived stem cells (sub-ADSCs).

### Cell population of the invention

In another aspect the invention relates to a substantially homogenous isolated population of stem cells, hereinafter "cell population of the invention", comprising a group of adult stem cells derived from fatty heart tissue of a mammal constitutively expressing GATA-4 and/or Cx43 (stem cells of the invention).

In a particular embodiment, the cell population of the invention comprises adult stem cells derived from fatty heart tissue of a mammal constitutively expressing GATA-4 and Cx43.

In another particular and preferred embodiment, the cell population of the invention comprises stem cells of the invention in which the constitutive expression of GATA-4 and/or Cx43 is maintained stable during its *in vitro* expansion.

In another particular embodiment, the cell population of the invention comprises stem cells of the invention which furthermore constitutively express β-MHC and/or SERCA-2.

In another particular embodiment, the cell population of the invention comprises stem cells of the invention which furthermore express one or more surface markers selected from CD29, CD44, CD59, CD90 and CD105. In a specific embodiment, the cell population of the invention has a significant expression of at least one, two, three, four, or preferably all the surface markers CD29, CD44, CD59, CD90 and CD105. In another particular embodiment, the stem cell of the invention is **characterized in that** it furthermore expresses the surface marker CD166. Therefore, in another specific embodiment, the stem cell of the invention has a significant expression of at least one, two, three, four, five, or preferably all the surface markers CD29, CD44, CD59, CD90, CD105 and CD166. As used herein, "significant expression" means that in said cell population, at least 30% of the cells show a signal for a specific cell surface marker determined by flow cytometry above the background signal, preferably 40%, 50%, 60%, 70% and more preferably 80%, 90%, 91%, 92%, 93%, 94%, 95%, 96%, 97%, 98%, 99% or 100%.

In another particular embodiment, the cell population of the invention comprises stem cells of the invention which do not express one, two, three or any of the surface markers selected from CD14, CD34, CD106 and CD117. In a specific embodiment, the cell population of the invention lacks significant expression of at least one, two, three, or preferably all the surface markers CD14, CD34, CD106 and CD117. In another particular embodiment, the stem cell of the invention is **characterized in that** it does not express (or it very weakly expresses) the surface marker VEGFR2. Therefore, in another specific embodiment, the stem cell of the invention lacks significant expression of at least one, two, three, four, or preferably all the surface markers CD14, CD34, CD106, CD117 and VEGFR2. As used herein, "lacks significant expression" means that, in said cell population, less than 30% of the cells show a signal for a specific cell surface marker in flow cytometry above the background signal, preferably less than 20%, 15% or 10%, more preferably less than 9%, 8%, 7%, 6%, 5%, 4%, 3%, 2% or 1%.

In another particular embodiment, the cell population of the invention comprises stem cells of the invention which are characterized, in addition to by their origin and by the constitutive expression of GATA-4 and/or Cx43, in that (i) they express all the surface markers CD29, CD44, CD59, CD90 and CD105, and (ii) they do not express any of the surface markers CD14, CD34, CD106 and CD117.

In another particular embodiment, the cell population of the invention comprises stem cells of the invention which are characterized, in addition to by their origin and by the constitutive expression of GATA-4 and/or Cx43, in that (i) they express all the surface markers CD29, CD44, CD90, CD105 and CD166 and (ii) they do not express any of the surface markers CD14, CD34, CD106, CD117 or VEGFR2.

In another particular embodiment, the cell population of the invention comprises stem cells of the invention which are characterized, in addition to by their origin and by the constitutive expression of GATA-4 and/or Cx43, in that (i) they express all the surface markers CD29, CD44, CD59, CD90, CD105 and CD166, and (ii) they do not express any of the surface markers CD14, CD34, CD106, CD117 or VEGFR2.

In another particular embodiment, the cell population of the invention comprises isolated adult stem cells derived from cardiac fatty (adipose) tissue of a mammal, preferably of a human being which a) constitutively express GATA-4 and/or Cx43; b) constitutively express β-MHC; c) express all the surface markers CD29, CD44, CD59, CD90 and CD105; and d) do not express any of the surface markers CD14, CD34, CD106 or CD117.

In another particular embodiment, the cell population of the invention comprises isolated adult stem cells derived from cardiac fatty (adipose) tissue of a mammal, preferably of a human being, which a) constitutively express GATA-4 and/or Cx43; b) constitutively express β-MHC; c) express all the surface markers CD29, CD44, CD90, CD105 and CD166; and d) do not express any of the surface markers CD14, CD34, CD106, CD117 or VEGFR2.

In another particular embodiment, the cell population of the invention comprises isolated adult stem cells derived from cardiac fatty (adipose) tissue of a mammal, preferably of a human being, which a) constitutively express GATA-4 and/or Cx43; b) constitutively express β-MHC; c) express all the surface markers CD29, CD44, CD59, CD90, CD105 and CD166; and d) do not express any of the surface markers CD14, CD34, CD106, CD117 or VEGFR2.

In another particular embodiment, the cell population of the invention comprises stem cells of the invention obtained from cardiac adipose tissue, e.g., epicardial or pericardial, of a mammal, such as a rodent, a primate, etc., preferably, of a human being. In a particular embodiment, the cell population of the invention comprises stem cells of the invention obtained from epicardial adipose tissue of a human being.

In another particular embodiment, the cell population of the invention comprises stem cells of the invention obtained from the stromal fraction of cardiac adipose tissue.

In another particular embodiment, the cell population of the invention comprises stem cells of the invention of an autologous, allogeneic or xenogeneic origin. In a particular and preferred embodiment, said cells are autologous and are isolated from the cardiac adipose tissue of the subject to whom they will be administered.

If desired, said cell population of the invention can be found in a cell bank for transplant. In a particular embodiment, said cell bank comprises a plurality of stem cells of the invention homozygotic for at least one or more genes of critical antigens, i.e., genes encoding histocompatibility antigens (e.g., an allele of the major histocompatibility complex (MHC) present in the human population). The cells of the invention homozygotic for one or more alleles of histocompatibility antigens compatible with the allele of the MHC of the subject in need of a cell transplantation or implantation can be selected from said bank.

If desired, the cell population of the invention can be kept frozen under conditions which neither affect nor compromise its viability after its reconstitution.

### Process for obtaining a composition comprising stem cells of the invention

In another aspect the invention relates to a process for obtaining a composition comprising adult stem cells derived from fatty heart tissue of a mammal constitutively expressing GATA-4 and/or Cx43, hereinafter process of the invention, comprising:
a) obtaining a cell suspension of a fatty heart tissue sample of a mammal;
b) separating the cells from said cell suspension;
c) cultivating said cells in a culture medium on a solid support under conditions which allow said cells to adhere to said solid support; and
d) recovering the adult stem cells derived from fatty heart tissue of a mammal constitutively expressing GATA-4 and/or Cx43.

The fatty heart tissue sample can be obtained from fatty epicardial tissue or from fatty pericardial tissue, preferably, from epicardial adipose tissue. Said fatty heart tissue sample is from a mammal, such as a rodent, a primate, etc., preferably a human being. Said fatty heart tissue sample of a mammal can be obtained by conventional methods known by persons of ordinary skill in the art. In a particular embodiment, said fatty heart tissue sample is extracted from the stromal fraction of the fatty tissue. A suitable source of fatty heart tissue is from the area close to the proximal right coronary artery or from the base of the heart around the aorta, from where the fatty heart tissue can be obtained in the context of routine heart surgery. Example 1 describes in detail a form of obtaining a human fatty heart (particularly epicardial) tissue sample.

The extracted fatty heart tissue sample is washed and cut into small fragments which are digested enzymatically (or by other conventional means) for the purpose of obtaining a cell suspension which is subjected to centrifugation, a cell pellet being obtained which is resuspended in a suitable medium (e.g., a culture medium comprising α-MEM medium supplemented with serum, glutamine and antibiotics) and seeded on a solid support (e.g., plastic, culture plate, culture flask, etc.) under conditions which allow the cells to adhere to said solid support (e.g., at 37°C and air atmosphere with 5% CO₂); once it is observed that the cells have been adhered (e.g., after approximately 24 hours depending on the culture conditions), the culture medium is removed and the adhered cells are washed before performing their *in vitro* expansion. To that end, the adhered cells (stem cells of the invention) are cultivated in the presence of a suitable culture medium (e.g., α-MEM medium supplemented with serum, glutamine and antibiotics) and under suitable conditions (e.g., 37°C, air atmosphere with 5% CO₂) and are maintained in culture in such conditions until pre-confluence (e.g., until a degree of confluence of approximately 80% is reached) periodically replacing all or part of the culture medium (e.g., every 3 or 4 days). The cells can be sub-cultivated repeatedly (passages) until reaching an amount of cell material (i.e., a minimum number of cells) which allows its analysis. In a particular embodiment, said cells are sub-cultivated at least two times (i.e., they are subjected to 2 passages), typically 3, 4, 5 or more times. In a specific embodiment, said cells were sub-cultivated 2 times (passage 2), whereas in another specific embodiment they were sub-cultivated 5 times, i.e., up to passage 5 (3-4 months), at the suitable dilutions. By operating in this manner, a cell density of 5-6x10³ stem cells of the invention/cm² can be obtained after being peeled off the solid support (e.g., culture plate, etc.). In both cases (passages 2 and 5), the expression of GATA-4 and Cx43 was analyzed, observing that the constitutive expression of said cardiospecific markers is maintained substantially stable throughout the *in vitro* expansion of said cells (stem cells of the invention). Examples 1 and 6 describe in detail obtaining, identifying, characterising and isolating stem cells of the invention from human fatty epicardial tissue.

The resulting composition comprising stem cells of the invention, obtainable according to the previously described process of the invention, is an additional aspect of this invention.

### Differentiated cells

In another aspect the invention relates to a method for obtaining differentiated cells comprising cultivating stem cells of the invention in a suitable specific differentiation medium. In a particular embodiment, said specific differentiation medium is a specific medium for the differentiation into the cardiomyogenic lineage. In another particular embodiment, said specific differentiation medium is a specific medium for the differentiation into the endothelial lineage. In another particular embodiment, said specific differentiation medium is a specific medium for the differentiation into adipogenic, osteogenic or chondrogenic. Said specific differentiation media are known by persons of ordinary skill in the art.

The differentiated cells obtainable from said cell differentiation method, hereinafter differentiated cells of the invention, are an additional aspect of the invention. In a particular embodiment, said differentiated cells of the invention are cardiomyocytes, osteocytes or chondrocytes.

In another aspect, the invention also relates to a composition comprising said differentiated cells of the invention and a suitable medium.

### Pharmaceutical composition

In another aspect the invention relates to a pharmaceutical composition, hereinafter pharmaceutical composition of the invention, comprising a therapeutically effective amount of said cell population of the invention, or of said composition comprising stem cells of the invention obtainable according to the process of the invention, or of said composition comprising differentiated cells of the invention, and a pharmaceutically acceptable vehicle.

As used herein, the term "therapeutically effective amount" relates to the amount of stem cells of the invention present in said cell population of the invention, or in said composition comprising stem cells of the invention obtainable according to the process of the invention, or of said composition comprising differentiated cells of the invention, which must contain the pharmaceutical composition of the invention, for being capable of producing the desired therapeutic effect; said therapeutically effective amount will generally be determined, among other factors, by the own characteristics of the cells and the desired therapeutic effect that is sought. The therapeutically effective amount of cells of the invention which must be administered will generally depend, among other factors, on the grade of the disease to be treated, on the own characteristics of the subject, on the affected area, etc. For this reason, the doses mentioned in this description must be taken into consideration only as a guideline for the person skilled in the art, who must adjust said dose depending on the previously described factors. As an illustrative and non-limiting example, the pharmaceutical composition of the invention can be administered as a single dose, containing approximately between 1x10⁵ and 1x10⁹, preferably between 1x10⁶ and 1x10⁸, more preferably between 1x10⁷ and 5x10⁷ stem cells of the invention, which can be partially or completely differentiated, or combinations thereof. The dose can be repeated, depending on the patient's condition and progression, in time intervals of days, weeks or months, which the specialist must establish in each case.

The stem cells of the invention contained in the cell population of the invention or in said composition comprising stem cells of the invention obtainable according to the process of the invention, as well as the differentiated cells of the invention contained in said composition, can be autologous, allogeneic or xenogeneic cells. In a particular and preferred embodiment, said cells are autologous and are isolated from the cardiac adipose tissue of the subject to whom they will be administered, thus reducing the potential complications associated with the antigenic and/or immunogenic responses to said cells.

If desired, the stem cells of the invention contained in the cell population of the invention or in said composition comprising stem cells of the invention obtainable according to the process of the invention, can be purified, as previously mentioned, using a selection of positive and/or negative cells by means of antibodies for the purpose of enriching the cell population to increase the efficacy, reduce the morbidity or facilitate the process.

According to the invention, the stem cells of the invention contained in the cell population of the invention or in said composition comprising stem cells of the invention obtainable according to the process of the invention, as well as the differentiated cells of the invention, can be administered to the patient without additional processing or following additional processes for purifying, stimulating or otherwise additionally changing the cells. For example, the stem cells of the invention obtained from a subject can be administered to another subject in need thereof after being cultivated before their administration. The cell population of the invention or said composition comprising stem cells of the invention obtainable according to the process of the invention, or said differentiated cells of the invention, can also be administered isolated from or together with other cell populations, for example, together with the remaining components of the stromal fraction of the cardiac adipose tissue. In a particular embodiment, the collection of cardiac adipose tissue will be done next to the patient's bed. Hemodynamic control can be used to monitor the patient's clinical condition. According to the invention herein described, the pharmaceutical composition of the invention can be administered to the patient shortly after the cardiac adipose tissue is extracted. For example, the pharmaceutical composition of the invention can be administered immediately after processing the cardiac adipose tissue to isolate the cell population of the invention or the composition comprising stem cells of the invention obtainable according to the process of the invention, and having placed it in a pharmaceutically suitable vehicle. In another embodiment, the delivery time will depend on the patient's availability and the time required to process the cardiac adipose tissue and isolate the cell population of the invention.

The term "pharmaceutically acceptable vehicle" relates to a vehicle which must be approved by a federal or state government regulatory agency or listed in the United States Pharmacopoeia or European Pharmacopoeia, or another generally recognized pharmacopoeia for its use in animals, and more specifically in humans.

The term "vehicle" relates to a diluent, coadjuvant, excipient or carrier with which the cells of the cell population of the invention or of said composition comprising stem cells of the invention obtainable according to the process of the invention must be administered; obviously, said vehicle must be compatible with said cells. Illustrative, non-limiting examples of said vehicle include any physiologically compatible vehicle, for example, isotonic solutions (e.g., sterile saline (0.9% NaCl), phosphate buffered saline (PBS), Ringer-lactate solution, etc.), optionally supplemented with serum, preferably with autologous serum; cell culture media (e.g., DMEM, etc.); or, alternatively, a solid, semisolid, gelatinous or viscous support means, such as collagen, collagen-glycosamino-glycan, fibrin, polyvinyl chloride, polyamino acids, such as polylysine, or polyornithine, hydrogels, agarose, silicone dextran sulfate. If desired, the support means can also, in specific embodiments, contain growth factors or other agents. If the support is solid, semisolid, or gelatinous, the cells can be introduced in a liquid phase of the vehicle which is subsequently treated such that it is converted into a more solid phase. In some embodiments of the invention in which the vehicle has a solid structure, said vehicle could be formed according to the shape of the injury.

If desired, the pharmaceutical composition of the invention can also contain, when necessary, additives to increase, control or otherwise direct the desired therapeutic effect of the cells, comprised in said pharmaceutical composition, and/or auxiliary substances or pharmaceutically acceptable substances, such as buffering agents, surfactants, cosolvents, preservatives, etc. It is also possible to add metal chelating agents. The stability of the cells in the liquid medium of the pharmaceutical composition of the invention can be improved by means of adding additional substances, such as, for example, aspartic acid, glutamic acid, etc. Said pharmaceutically acceptable substances which can be used in the pharmaceutical composition of the invention are generally known by persons of ordinary skill in the art and are normally used in the preparation of cell compositions. Examples of suitable pharmaceutical vehicles are described, for example, in "Remington's Pharmaceutical Sciences", by E.W. Martin. Additionally information about said vehicles can be found in any pharmaceutical technology manual (Galenic Pharmacy).

The pharmaceutical composition of the invention will contain a therapeutically effective amount of the cell population of the invention or of said composition comprising stem cells of the invention obtainable according to the process of the invention, or of said composition comprising differentiated cells of the invention, preferably a substantially homogenous cell population of the invention, after being isolated and expanded, together with the suitable vehicle in the appropiate amount to provide the correct dosage form to the subject.

The pharmaceutical composition of the invention will be formulated according to the chosen dosage form. The formulation will be adjusted to the mode of administration. In a particular embodiment, the pharmaceutical composition of the invention is prepared in a liquid dosage or gel mode, for example, in the form of a suspension, to be injected or perfused to the subject in need of treatment. Illustrative and non-limiting examples include the formulation of the pharmaceutical composition of the invention in a sterile suspension with a pharmaceutically acceptable excipient, such as an isotonic solution, for example, phosphate buffered saline (PBS), or any other suitable pharmaceutically acceptable vehicle, for the administration to a subject parenterally, e.g., a human being, preferably intravenously, intraperitoneally, subcutaneously, etc., although other alternative administration routes are possible.

The administration of the pharmaceutical composition of the invention to the subject in need thereof will be performed by conventional means. In a specific application, said pharmaceutical composition can be administered to said subject intravenously using suitable devices, such as syringes, catheters, trocars, cannulas, etc. In all cases, the pharmaceutical composition of the invention will be administered using the equipment, apparatuses and devices suited to the administration of cell compositions and known by the person skilled in the art.

In a specific embodiment, the pharmaceutical composition of the invention is administered intravenously and includes an intravenous administration through standard devices, for example, a standard peripheral intravenous catheter, a central venous catheter or a pulmonary artery catheter, etc. The flow of the cells can be controlled by serially inflating or deflating distal and proximal globes located in the patient's vasculature.

In another particular embodiment, the direct administration of the pharmaceutical composition of the invention to the site sought to be benefited can be advantageous. Therefore, if desired, the pharmaceutical composition of the invention can be administered (implanting, transplanting, etc.) directly to the desired organ or tissue applying it directly (e.g., by injection, etc.) on the external surface of the affected organ or tissue by means of inserting a suitable device, e.g., a suitable cannula, catheter, etc., by arterial or venous perfusion (including retrograde flow mechanisms) or by other means mentioned in this description or known in the art. In a preferred embodiment, the pharmaceutical composition of the invention will be directly administered in the damaged area of the myocardium by means of, for example, intracoronary injection or by transmyocardial injection by means of a catheter. Catheters designed for the release of active ingredients specifically in a damaged area of the heart, particularly, in the infarcted area, have been described (see, for example, United States patents US 6, 102, 926, US 6, 120, 520, US 6,251,104, US 6,309,370, US 6,432,119 and US 6,485,481). The administration system used can include, for example, an apparatus for the intracardiac administration of alternative medicines, including a sensor for intracardiac positioning and a release system for administering the desired active ingredient in the desired amount in the position of the sensor.

If desired, the pharmaceutical composition of the invention can be stored until the time it is applied by means of conventional processes known by persons of ordinary skill in the art. This pharmaceutical composition can also be stored together with additional medicines useful in the post-myocardial infarction treatment and/or congestive heart failure, in an active form comprising a combined therapy. For short-term storage (less than 6 hours), the pharmaceutical composition of the invention can be stored at room temperature or under said temperature in a sealed container, supplementing it or not with a nutrient solution. The mid-term storage (less than 48 hours) is preferably at 2-8°C, and the pharmaceutical composition of the invention will include an iso-osmotic buffered solution and in a container made of or coated with a material which prevents cell adhesion. The more long-term storage is preferably performed by means of suitable cryopreservation and storage in conditions which promote the retention of cell function.

In a specific embodiment, the pharmaceutical composition of the invention is used in combined therapy. In a particular embodiment, said pharmaceutical composition is administered in combination with an additional pharmaceutical composition for the post-myocardial infarction treatment and/or congestive heart failure. Therefore, the stem cells of the invention contained in the cell population of the invention can be used as a single treatment or combined with other conventional treatments for the treatment of cardiovascular disease, and particularly of ischemic heart disease, such as, for example, for performing a coronary bypass, an angioplasty (with or without stents), the administration of angiogenesis promoters, the implantation of a ventricular assist device, the administration of thrombolytic agents, antiplatelet-aggregating agents (acetylsalicylic acid and/or clopidogrel), antihypertensive agents (angiotensin converting enzyme inhibitors (ACE inhibitors), angiotensin I receptor antagonists (ARA-II), β receptor blockers, diuretics, antilipidemic agents, digoxin, nitrates and/or calcium antagonists.

In a particular embodiment, the combined therapy is administered to a subject with an ischemic heart disease, particularly to a patient who has suffered a myocardial infarction and/or suffers congestive heart failure which does not respond to conventional treatments.

The pharmaceutical composition of the invention can be used in a combined therapy with additional medication useful in the post-myocardial infarction treatment and/or congestive heart failure, as previously described. Said additional medicinal products can form part of the same pharmaceutical composition or they can alternatively be supplied in the form of a separate composition for the simultaneous or successive (sequential in time) administration with respect to the administration of the pharmaceutical composition of the invention. In a specific embodiment, said additional pharmaceutical composition is administered simultaneously or sequentially to the pharmaceutical composition comprising the cell population of the invention, spaced out in time, in any order, i.e., the pharmaceutical composition of the invention can be administered first, then the other additional medicines or other pharmaceutical composition for the treatment of an ischemic heart disease, or the other additional medicines or other pharmaceutical composition for the treatment of an ischemic heart disease can be administered first and then the pharmaceutical composition of the invention. Either of these two components can alternatively be mixed in the same composition and administered together. In another alternative embodiment, said pharmaceutical composition of the invention and other additional medicines or other pharmaceutical composition for the treatment of an ischemic heart disease are simultaneously administered.

The patients can be monitored before and during the administration of the pharmaceutical composition of the invention. After the administration of the pharmaceutical composition, the patients may require an approximate period of 24 hours of monitoring in case adverse effects occur. Follow-up studies are recommended to evaluate functional improvements.

### Biomaterial comprising the cell population of the invention

In another aspect the invention relates to a biomaterial, hereinafter biomaterial of the invention, comprising said cell population of the invention, said composition comprising stem cells of the invention obtainable according to the process of the invention, or said composition comprising differentiated cells of the invention, or said pharmaceutical composition of the invention.

Tissue engineering consists of transplanting in the damaged tissues biomaterials which are made up of biocompatible structures suitable for their implantation in the organism which have been coated with cells with the capability of adhering and proliferating. Said structures can be, among others: sutures, matrices, membranes, foams, gels and ceramics. Different materials are known which have been used in the construction of matrices and other biocompatible structures, including: inorganic materials, for example, metals; natural polymers such as fibrin or alginates; synthetic polymers such as polyhydroxy acids, for example, polyglycolic acid (PGA) and copolymers thereof (e.g., poly (lactic-co-glycolic acid) (PLGA), etc.). In a preferred embodiment, said polymers are biodegradable, such that they degrade over time and the polymeric structure is completely replaced by cells. In a particular embodiment, said biomaterial of the invention comprises, or is made up of, a biocompatible structure comprising one or more biodegradable polymers and the cell population of the invention, or a composition comprising differentiated cells of the invention, or a pharmaceutical composition of the invention.

In another particular embodiment, said polymeric structure can be coated with bioactive molecules, i.e., with molecules capable of interacting specifically with the cells, or with another polymer with better adherence properties for the purpose of increasing the degree of adherence and proliferation of the cells.

### Use of the cell population of the invention or of said composition comprising stem cells of the invention obtainable according to the process of the invention, or of said composition comprising differentiated cells of the invention, in the preparation of a medicine for the treatment of pathologies

The inventors have observed that the cell population of the invention as well as said composition comprising stem cells of the invention obtainable according to the process of the invention, have a good cardiomyogenic potential, better than that of other populations of stem cells of different origins already described, therefore the cell population of the invention as well as said composition comprising stem cells of the invention obtainable according to the process of the invention, are cell-based reagents potentially useful in cardiac tissue regeneration and/or in the treatment of situations in which there is a loss of functional myocardial tissue (ischemic heart disease), for example, in patients who have suffered one or more myocardial infarctions or in patients who have developed congestive heart failure as well as in the stimulation of angiogenesis in situations in which it is appropriate to stimulate it.

Therefore, in another aspect the invention relates to the use of a cell population of the invention, or of said composition comprising stem cells of the invention obtainable according to the process of the invention, or of said composition comprising differentiated cells of the invention, in the preparation of a pharmaceutical composition for cardiac tissue regeneration, or in the preparation of a pharmaceutical composition for the treatment of an ischemic heart disease, or in the preparation of a pharmaceutical composition for the post-myocardial infarction treatment, or for the treatment of congestive heart failure, or in the preparation of a pharmaceutical composition to stimulate angiogenesis.

In another aspect the invention relates to the cell population of the invention, or of said composition comprising said stem cells of the invention, or of said composition comprising differentiated cells of the invention, for cardiac tissue regeneration, or for the treatment of an ischemic heart disease, or for the post-myocardial infarction treatment, or for the treatment of congestive heart failure and/or to stimulate angiogenesis.

To regenerate cardiac tissue, or for the treatment of an ischemic heart disease, post-myocardial infarction, or congestive heart failure, or to stimulate angiogenesis, the pharmaceutical composition of the invention comprising a cell population of the invention or a composition comprising stem cells of the invention obtainable according to the process of the invention, or of said composition comprising differentiated cells of the invention, a therapeutically effective amount of said pharmaceutical composition is administered to the subject in need of treatment. As previously mentioned, the stem cells of the invention (present in said cell population of the invention), are derived from cardiac adipose tissue and constitutively express GATA-4 and/or Cx43, preferably both. The Cx43 protein is one of the main proteins in the gap junctions electrically connecting the cardiomyocytes; therefore the fact that said stem cells of the invention constitutively express Cx43 suggests good electric coupling with the pre-existing cardiac tissue after the transplantation of said cell population comprising stem cells of the invention.

The stem cells of the invention, the cell population of the invention, or said composition comprising stem cells of the invention obtainable according to the process of the invention, or said composition comprising differentiated cells of the invention, can be used to obtain a suitable number of cells capable of regenerating the ischemic cardiac tissue or to improve the functionality of the heart after one or more myocardial infarctions. In a particular embodiment, said improvement is due to the differentiation of the stem cells of the invention present in said cell population of the invention into cardiomyocytes, smooth muscle and/or vascular endothelial tissue. As previously described, the administration of the pharmaceutical composition of the invention to the subject in need thereof can be done by conventional means. In a specific embodiment, said pharmaceutical composition can be administered to the subject in need thereof directly in the damaged area of the myocardium, such as, for example, by means of intracoronary injection or by transmyocardial injection by means of catheter.

The stem cells of the invention, the cell population of the invention, or said composition comprising stem cells of the invention obtainable according to the process of the invention, or said composition comprising differentiated cells of the invention, can be used to obtain a suitable number of cells capable of stimulating angiogenesis in pathological situations in which it can be necessary.

### Method for cardiac tissue regeneration, or for the treatment of an ischemic heart disease, or for the post-myocardial infarction treatment, or for the treatment of congestive heart failure, or to stimulate angiogenesis based on the use of stem cells of the invention or of differentiated cells of the invention

The invention also relates to cardiac tissue regeneration, to the treatment of pathological situations in which there is a loss of functional myocardial tissue (e.g., ischemic heart disease), for example, in subjects (patients) who have suffered one or more myocardial infarctions or in patients who have developed congestive heart failure. Likewise, the invention also relates to the stimulation of angiogenesis in situations in which it is appropriate or advisable.

Therefore, in another aspect the invention relates to a method for cardiac tissue regeneration, or for the treatment of an ischemic heart disease, or for the post-myocardial infarction treatment, or for the treatment of congestive heart failure, or for the stimulation of angiogenesis, comprising the administration to a subject in need thereof of a therapeutically effective amount of stem cells of the invention, or of a composition comprising stem cells of the invention obtainable according to the process of the invention, or of said composition comprising differentiated cells of the invention, or of a pharmaceutical composition of the invention.

As previously mentioned, to regenerate cardiac tissue, or for the treatment of an ischemic heart disease, post-myocardial infarction, congestive heart failure, or to stimulate angiogenesis, said stem cells of the invention, said composition comprising stem cells of the invention obtainable according to the process of the invention, said composition comprising differentiated cells of the invention, or said pharmaceutical composition of the invention, are administered to the subject in need of treatment in a therapeutically effective amount, by conventional methods known by persons of ordinary skill in the art (e.g., by means of direct administration to the damaged area of the myocardium by intracoronary injection, transmyocardial injection, etc.).

### Kit

In another aspect the invention relates to a kit comprising a stem cell of the invention, a cell population of the invention, a composition comprising stem cells of the invention obtainable according to the process of the invention or said composition comprising differentiated cells of the invention. The characteristics of said stem cells and cell population of the invention, as well as of said composition comprising stem cells of the invention obtainable according to the process of the invention, and of said composition comprising differentiated cells of the invention have already been previously described. The stem cells of the invention, present in the cell population of the invention or in said composition comprising stem cells of the invention obtainable according to the process of the invention, as with said differentiated cells of the invention present in said composition, which form part of said kit can be of an allogeneic or xenogeneic origin.

Said kit can be used for diagnostic purposes and/or for *in vitro* research, therefore, for such purposes, if desired, the stem cells of the invention can be immortalized such that they are capable of indefinitely expanding.

Therefore, in a particular embodiment, the stem cells of the invention are subjected to a process of immortalization, for example, to a process of reversible immortalization, for the purpose of obtaining immortalized stem cells, preferably, reversibly immortalized stem cells of the invention. In this sense, as used herein, the term "immortalization" or "immortalized" relates to a cell, or to a process for the creation of a cell, which will indefinitely proliferate in culture without entering in senescence. According to the present invention, immortalization relates to a process whereby a cell culture is transformed such that the cells behave in some aspects like tumor cells, particularly in relation to the proliferative characteristics of tumor cells. Thus, an "reversibly immortalized cell" relates to a cell which, at a given time, is in an immortalized state but can be returned to a non-immortalized state at a later time using a reverse immortalization process. In a particular embodiment, the stem cells of the invention are reversibly immortalized by means of a process comprising: (a) transforming stem cells of the invention with a vector comprising a "removable polynucleotide" containing an oncogene (or a combination of oncogenes), for the purpose of obtaining immortalized stem cells of the invention; (b) growing said immortalized stem cells of the invention; and (c) selecting those clonal cell lines of immortalized stem cells of the invention which maintain the functional properties of the cells of the invention; if desired, the oncogene (or combination of oncogenes) can be removed from the immortalized stem cells of the invention. By way of illustration, cell populations can be immortalized by means of individual overexpression or in combination with some oncogenes, such as the SV40 large T-antigen, the telomerase catalytic subunit, Bmi-1, etc. The overexpression of these oncogenes could be reversed by means of flanking with recombinase targets (e.g., introducing Cre recombinase which recognizes loxP targets, etc.), and, furthermore by adding the suicide gene of thymidine kinase which would allow the destruction of immortalized cells.

### Method for evaluating in vitro cell response to biological or pharmacological agents

In another aspect the invention relates to a method for evaluating *in vitro* cell response to a biological or pharmacological agent, comprising contacting said agent with a cell population of the invention, comprising a plurality of stem cells of the invention, or with a composition comprising stem cells of the invention obtainable according to the process of the invention, optionally differentiated into a specific cell type, or with said composition comprising differentiated cells of the invention, and evaluating the effects of said agents on said cell population in culture.

By way of illustration, cell response to a biological or pharmacological agent can be evaluated *in vitro* by means of a process comprising: (a) isolating a cell population of the invention or a composition comprising stem cells of the invention obtainable according to the process of the invention, from an individual or from a group of individuals; (b) optionally, differentiating all or part of the stem cells of the invention present in said cell population, or in said composition comprising stem cells of the invention obtainable according to the process of the invention, into a specific cell type or lineage; (c) expanding *in vitro* the cells resulting from step (a) or (b) in culture; (d) optionally, differentiating the cells expanded in step (c) into a specific cell type; (e) contacting the cell population resulting from step (c) or (d) in culture with one or more biological or pharmacological agents; and (f) evaluating the effects of said agents on the cell population in culture.

In a particular embodiment, the stem cells of the invention, present in the cell population of the invention or in the composition comprising stem cells of the invention obtainable according to the process of the invention, are differentiated into cardiomyocytes. The differentiation step can take place either after the isolation of the cell population of the invention [step (b)] or after its *in vitro* expansion [step (d)].

### Process for obtaining growth factors and/or cytokines

In another aspect the invention relates to a process for obtaining growth factors and/or cytokines comprising cultivating stem cells of the invention, or differentiated cells of the invention, under conditions suitable for the expression and production of said growth factors and/or cytokines, and, if desired, separating said growth factors and/or cytokines. Said conditions are known by persons of ordinary skill in the art or can be easily deduced by a person skilled in the art in view of the information contained in this description.

The following examples illustrate the invention and must not be considered in a limiting sense thereof.

### EXAMPLE 1

### Cell isolation and cultivation of populations of sub-ADSC and epi-ADSC adult stem cells

Two populations of substantially homogenous stem cells were isolated from samples of human fat of epicardial and subcutaneous origin of one and the same individual and both their phenotype and the basal expression of cardiospecific markers were compared.

### Materials and Methods

### Obtaining samples of epicardial and subcutaneous fat

The samples of epicardial and subcutaneous fat were obtained from 4 patients (P1, P2, P3 and P4) in routine heart surgery after having received the informed consent, a sample of each type of fat being extracted from each of them. During the heart surgery, first the cutaneous and subcutaneous tissue was dissected until exposing the sternum. A fragment (around 2 to 5 g) of fat was obtained from the thoracic subcutaneous tissue exposed in this process using surgical clamps. Then median sternotomy and dissection of the pericardium is performed with the subsequent exposure of the heart. The epicardial adipose tissue (around 0.5 to 2 g) was obtained from the base of the heart around the aorta. This adipose tissue was selected by means of surgical clamps and resected using usual surgical scissors. The study was approved by the Ethics Committee of the Santa Creu i Sant Pau Hospital.

### Isolation of the epi-ADSC and sub-ADSC cell populations from the samples of epicardial and subcutaneous fat

Both types of fat samples (epicardial and subcutaneous) were washed repeatedly with PBS buffer (Gibco Invitrogen Corp.) and cut into small fragments using a scalpel after dissecting the blood vessels present.

Then, the fragments of adipose tissue were enzymatically digested with a solution of type II collagenase at 0.05 % in α-MEM (Gibco Invitrogen Corp) for 30 minutes at 37°C under stirring. The reaction was stopped by adding α-MEM medium supplemented with 10% fetal bovine serum (FBS), 1 mM L-glutamine (Gibco Invitrogen Corp) and 1% penicillin-streptomycin (Gibco Invitrogen Corp). Next the suspension was centrifuged at 1,200 g for 10 minutes at room temperature. The supernatant was discarded, the pellet was resuspended with α-MEM complete culture medium supplemented with 10% FBS, 1 mM L-glutamine and 1% penicillin-streptomycin and was seeded in culture vessels (at 37°C and air atmosphere with 5% CO₂). After 24 hours the culture medium was removed and the adhered cells were washed with PBS.

### In vitro expansion of the previously isolated cells

The adhered cells were cultivated in the presence of α-MEM supplemented with 10% FBS, 1 mM L-glutamine and 1% penicillin-streptomycin at 37°C under air atmosphere with 5% CO₂. The cells were maintained in culture under the same conditions until a degree of confluence of approximately 80% (pre-confluence) was reached, replacing the culture medium every 3 or 4 days. The cells were repeated sub-cultivated upon reaching pre-confluence until passage 5 (3-4 months), at a 1:3 dilution, which corresponds to a density of 5-6x10³ cells/cm², after being peeled off the culture plate by means of a solution of trypsin/EDTA.

### Results

A population of adult epicardial adipose tissue-derived stem cells and a population of adult subcutaneous adipose tissue-derived stem cells from the same individual were selected and expanded in culture *in vitro.* Figure 1A shows the extracted fractions of epicardial and subcutaneous fat. The morphology of the human epicardial adult adipose tissue-derived stem cells (epi-ADSC) is shown in Figure 1B, whereas the morphology of the human adult subcutaneous adipose tissue-derived stem cells (sub-ADSC) is shown in Figure 1C.

### EXAMPLE 2

### Immunophenotypic characterization of the population of epi-ADSC adult stem cells

The expression of different surface markers was analyzed by means of flow cytometry for the purpose of characterising the population of epicardial adipose tissue-derived stem cells (epi-ADSC) and the results obtained were compared with those of the cell population isolated from subcutaneous adipose tissue (sub-ADSC).

### Materials and Methods

### Flow cytometry

Immunophenotypic analysis by means of flow cytometry was performed in four cell populations derived from epicardial adipose tissue (epi-ADSC cells) isolated from four samples from patients identified as P1, P2, P3 and P4, and they were compared to a representative sample of sub-ADSC cells. The P1 and P2 epi-ADSC cells were characterized after being cultivated for 3-4 weeks (low passage), whereas the P3 and P4 epi-ADSC cells were characterized after being cultivated for 9-12 weeks (high passage).

The cells were washed with PBS at 4°C and were peeled off the plate with 0.05% trypsin/EDTA (Gibco) for 5 minutes in culture conditions. Once the action of the trypsin was blocked, the cells were centrifuged at 1,400 rpm for 5 minutes in cool conditions. The cells were maintained in cooled staining buffer (1X PBS with 1% FCS) during the entire staining process. The staining was carried out by means of antibodies specific for different surface antigens: CD3, CD9, CD10, CD11B CD13, CD14, CD15, CD16, CD18, CD19, CD28, CD29, CD31, CD34, CD36, CD38, CD44, CD45, CD49a, CD49d, CD49e, CD49f, CD50, CD51, CD54, CD55, CD56, CD58, CD59, CD61, CD62e, CD621, CD62p, CD71, CD90, CD95, CD102, CD104, CD105, CD106, CD117, CD133/2, CD59, CD235a, HLAI, HLAII, NGFR and β2-microglobulin (all of which are from Serotec).

Two fluorophors were used to show the presence of said antigens in the cell membrane, fluorescein isothiocyanate (FITC) and phycoerythrin (PE), diluted 1/50 in staining buffer for 20 minutes protected from the light and at 4°C. The samples were analyzed in a Coulter EPICS XL cytometer and by means of specific software (FCS Express 3 software).

### Results

Figure 2 shows the positive results obtained of the immunophenotypic profile of the epi-ADSC samples. The statistical parameter used for the analysis was "% positive". This parameter measures the percentage of the cells accepted in the acquisition formula which it considers positive with respect to the control. The FCS Express program statistically performs a numerical subtraction and considers as a positive result a value greater than 30% of the "percentage of cells positive" parameter.

Accordingly, the results of the analysis of the immunophenotypic profile by means of flow cytometry show that more than 90% of the markers studies have similar expression in both cell populations (epi-ADSC and sub-ADSC). Specifically, the population of epi-ADSC cells is very positive for CD9, CD29, CD44, CD51, CD54, CD55, CD59, CD90, CD105, HLA-I and β2-microglobulin, as in the case of the population of sub-ADSC cells (Table 1). The remaining negative specific markers of sub-ADSC are also negative for epi-ADSC cells. Nevertheless, a difference has been detected in relation to the expression of cell surface marker CD54, which is positive in the case of epi-ADSC cells and negative in sub-ADSC cells.

**Table 1**

| Results of the expression of the surface markers analyzed by flow cytometry in the different samples of epi-ADSC cells compared to a representative sample of sub-ADSC cells | | | | | |
|---|---|---|---|---|---|
| | | **epi-ADSC** | | | |
| **% Positive Markers** | **sub-ADSC** | **P1** (low passage) | **P2** (low passage) | **P3** (high passage) | **P4** (high passage) |
| **CD9** | 70 | 77.95 | 88.11 | 72.63 | 76.69 |
| **CD29** | 71.22 | 70.86 | 81.88 | 80.1 | 72.44 |
| **CD44** | 48.74 | 81.34 | 88.33 | 88.66 | 73.47 |
| **CD49b** | 28.44 | 50.93 | 70.45 | 59.98 | 58.53 |
| **CD49c** | 29.22 | 47.95 | 61.83 | 49.95 | 55.56 |
| **CD51** | 52.28 | 83.94 | 88.76 | 65.49 | 61.57 |
| **CD54** | 14.53 | 42.96 | 60.09 | 72.33 | 50.94 |
| **CD55** | 69.95 | 43.36 | 68.92 | 72.59 | 47.58 |
| **CD59** | 99.57 | 96.84 | 94.34 | 95.06 | 96.29 |
| **CD90** | 94.09 | 66.02 | 73.37 | 73.33 | 72.32 |
| **CD105** | 95.03 | 63.55 | 70.1 | 85.66 | 82.18 |
| **HLA-I** | 81.89 | 70.58 | 72.36 | 78.81 | 74.84 |
| **β2 microglob.** | 92.31 | 75.37 | 76.43 | 79.17 | 72.37 |

| | | | | | |
|---|---|---|---|---|---|
| [NOTE: Only those markers having a positive result (expression of greater than 30%) are shown] | | | | | |

### EXAMPLE 3

### Comparative study of the gene expression of cardiospecific markers in populations of sub-ADSC and epi-ADSC stem cells

The basal expression of the cardiospecific markers β-MHC, GATA-4, Nkx2.5, cardiac troponin I (cTnI), SERCA-2, sarcomeric α-actinin and connexin-43 was analyzed by means of the real-time RT-PCR technique.

### Materials and Methods

### Real-time RT-PCR

The total RNA of the isolated cells was extracted with the *QuickPrep Total RNA Extraction* kit (Amersham) according to the manufacturer's instructions. The cDNA was synthesized using 2 µg of total RNA by means of a reverse transcription reaction using the Script One-Step RT-PCR kit with random hexamers (Bio-Rad Laboratories) at 50°C during 10 minutes. Once the cDNA was obtained, all the PCR reactions were prepared with primers marked with FAM®, specific for all the studied genes, in the *TaqMan Universal PCR master mix* kit (Applied Biosystems). The primers used were: GATA-4 (Hs00171403_m1), Nkx2.5 (Hs00231763_m1), sarcomeric α-actinin (Hs00241650_m1), β-MHC (Hs00165276_m1), connexin-43 (Hs00748445_s1), SERCA-2 (Hs00544877_m1), cTnI (Hs00165957_m1) and GAPDH (Hs99999905_m1) (Applied Biosystems) and the reaction conditions were: 2 minutes at 50°C, 10 minutes at 95°C and 40 cycles of 15 seconds at 95°C and 1 minute at 60°C for all the primers. The amplification was finally analyzed by means of the *ABI Prism 7000 Sequence Detection* system (Applied Byosistems). To quantify the gene expression, the obtained data was analyzed with the *ABI Prism 7000 SDS software* program. All the samples were analyzed in duplicate. The Ct (Δ Ct) comparison method was used; Ct is the PCR cycle in which the first increase in fluorescence above the basal level is detected) to calculate the relative expression of each gene analyzed using the constitutive expression gene GAPDH as endogenous reference control.

### Results

As is shown in Table 2 and in Figure 3A, a statistically significant increase of the gene levels for the cardiac transcription factor GATA-4 in the population of epi-ADSC cells in comparison with the sub-ADSC cells extracted from the same individual (p<0.001) was detected at passage 2. In contrast, significant differences were not detected in relation to the gene expression of the remaining cardiospecific markers (α-actinin, β-MHC, cTnI, Cx43, SERCA-2 and Nkx2.5) between the two types of cell populations studied (Figure 3A). It should be pointed out that at passage 5 the difference of expression of GATA-4 increases (p<0.001) and a significant difference is also detected with respect to the transcript levels for Cx43 (p=0.031) (Figures 3B and 3C). These results indicate that the population of epicardial fat-derived stem cells (epi-ADSC) is more committed towards cardiac lineage in comparison with the population of cells from subcutaneous fat (sub-ADSC) from the same individual.

**Table 2**

| Quantification of the gene expression levels | | | | |
|---|---|---|---|---|
| **Amplified gene** | **Passage 2** | | **Passage 5** | |
| | **epi-ADSC** | **sub-ADSC** | **epi-ADSC** | **sub-ADSC** |
| **Cx43** | 0.92 ± 0.1 | 0.77 ± 0.1 | 1.17 ± 0.2 | 0.78 ± 0.2 |
| **α-actinin** | 0.92 ± 0.2 | 0.77 ± 0.2 | 1.03 ± 0.1 | 0.74 ± 0.2 |
| **cTnI** | 0 | 0 | 0.99 ± 1.2 | 0.35 ± 0.2 |
| **β-MHC** | 0 | 0 | 0.90 ± 0.3 | 0.62 ± 0.4 |
| **GATA-4** | 0.88 ± 0.1 | 0.08 ± 0.2 | 1.07 ± 0.2 | 0 |
| **Nkx2.5** | 0 | 0 | 0 | 0 |
| **SERCA-2** | 0.90 ± 0.1 | 0.88 ± 0.26 | 0.92 ± 0.6 | 0.66 ± 0.2 |

### EXAMPLE 4

### Comparative study at the protein level of the expression of cardiospecific markers in the epi-ADSC cells and in the sub-ADSC cells

The basal expression of the cardiospecific markers β-MHC, GATA-4, Nkx2.5, cardiac troponin I, SERCA-2, sarcomeric α-actinin and connexin-43 was analyzed at the protein level by means of immunofluorescence and Western blot techniques.

### Materials and Methods

### Western Blot

The cells were washed repeatedly with Cold PBS buffer and were homogenized in lysis buffer [25 mM Tris pH 7.6, 150 mM NaCl, 1 mM EDTA (ethylenediaminetetraacetic acid), 1 mM EGTA (ethyleneglycoltetraacetic acid), 1% SDS (sodium lauryl sulfate), 1 mM PMSF (phenylmethyl sulfonyl fluoride), 1 µg/ml of aprotinin and 1 µg/ml of leupeptin] for 30 minutes at 4°C. The SDS-insoluble fraction was obtained by means of centrifugation at 13,000 rpm for 10 minutes at 4°C. The protein concentration of the total extracts was determined by means of the *Bio-Rad DC protein* kit (BioRad) using BSA as standard. 50 µg of protein were transferred to nitrocellulose membranes with a pore diameter of 0.45 mm and were developed with antibodies specific for the different cardiospecific markers analyzed: Cx43 (1/100) (BD Transduction Laboratories), GATA-4 (1/100), SERCA-2 (1/100), anti-cardiac troponin I (cTnI) (1/100) (the former 3 from Santa Cruz Biotechnology), β-MHC. (1/10) (Chemicon) and sarcomeric α-actinin (1/100) (Sigma)). A chemiluminescent detection system was used to view the corresponding specific bands (Pierce) according to the manufacturer's instructions.

### Immunofluorescence

The cells, cultivated in 35 mm plates with a 0.17 mm glass bottom, special for their study by means of confocal microscopy (FluoroDish, WPI Inc.), were washed repeatedly with PBS buffer and fixed with 4% PFA (paraformaldehyde) prepared in PBS for 15 minutes at room temperature. Then, the cells were permeabilized with 1% BSA (bovine serum albumin)/0.1% saponin in PBS for 30 minutes at room temperature. The cells were finally incubated with antibodies specific for the different cardiospecific markers analyzed: Cx43 (1/100) (BD Transduction Laboratories), GATA-4 (1/100), SERCA-2 (1/100), cardiac troponin I (cTnI) (1/100) (Santa Cruz Biotechnology), β-MHC. (not diluted) (Chemicon) and sarcomeric α-actinin (1/100) (Sigma)) and were finally analyzed under a fluorescence confocal microscope (Leica).

### Results

The results of the study of the expression of said markers at the protein level complement the results obtained in the analysis of the gene expression thereof.

It was thus observed that, in the absence of additional cardiogenic stimuli or factors in the culture medium, only the epi-ADSC cells expressed Cx43 and GATA-4 both at passage 2 and at passage 5 (Figures 4A and 4B). An increase of the expression levels of Cx43 and GATA-4 in the epi-ADSC cells was also observed throughout their *in vitro* expansion (from passage 2 to passage 5).

The absence of expression, at the protein level, of the calcium SERCA-2 pump and of β-MHC in both cell populations at passage 2 should be pointed out. However, an increase in the expression of β-MHC and SERCA-2 in both cell populations (Figures 4 and 5) was detected at passage 5, the epi-ADSC cells have an increase in the expression of β-MHC greater than that observed in the sub-ADSC cells from one and the same individual (Figure 4).

The existence of this differential expression at the protein level may be due to differences in the regulation mechanisms of the transcript translation process or the process for translating mRNA into a mature protein between the two cell populations studied.

In relation to the remaining genes/proteins analyzed, there is a high degree of coincidence between the results obtained by means of real-time RT-PCR and the Western blot and immunofluorescence experiments.

### EXAMPLE 5

### Analysis of the capability for differentiation of stem cells derived from fatty epicardial tissue (epi-ADSC)

The capability for differentiation of the stem cells derived from epicardial fat (epi-ADSCs) into adipogenic, osteogenic and chondrogenic lineage was analyzed.

### Materials and Methods

### Differentiation of epi-ADSC cells into osteogenic, adipogenic and chondrogenic lineages

Specific differentiation protocols were used for each of the cell lineages: osteogenic differentiation, adipogenic differentiation and chondrogenic differentiation.

### ○ Osteogenic Differentiation

The induction of differentiation into osteocytes was performed following a protocol already established for subcutaneous adipose tissue-derived stem cells and for other cell types (Zuk et al., 2002. Human adipose tissue is a source of multipotent stem cells. Mol Biol Cell. 2002 Dec; 13(12):4279-95).

After maintaining the cells for 1 week in culture (DMEM medium, 2 mM L-glutamine (Gibco), 100 U/mL of penicillin/streptomycin (Gibco), the differentiation into osteocytes was induced by means of cultivation for two weeks in a specific differentiation medium made up of: DMEM (BE12-614F Cambrex, Biowhitaker), 10% FBS (5253 Linus Cultek), 2 mM L-glutamine (Gibco), 100 U/mL of penicillin/streptomycin (Gibco), 100 nM dexamethasone (Sigma), 50 µM ascorbic acid-2-phosphate (Sigma) and 10 mM beta-glycerophosphate (Sigma).

### ○ Adipogenic Differentiation

The induction of differentiation into adipocytes was performed following a protocol already established for subcutaneous adipose tissue-derived stem cells and for other cell types (Zuk et al., 2002, cited above; Awad et al., 2003. Effects of transforming growth factor beta1 and dexamethasone on the growth and chondrogenic differentiation of adipose-derived stromal cells. Tissue Eng. 2003 Dec; 9(6):1301-12).

After maintaining the cells for 1 week in culture in DMEM medium, 2 mM L-glutamine (Gibco), 100 U/mL of penicillin/streptomycin (Gibco), the differentiation into adipocytes was induced by means of the alternation of two culture media (Medium A and Medium B), the composition of which is specified below:
Medium A: DMEM (BE12-614F Cambrex, Biowhitaker), 10% FBS (5253 Linus Cultek), 2 mM L-glutamine (Gibco), 100 U/mL of penicillin/streptomycin (Gibco), 1 µM dexamethasone (Sigma), 0.2 mM indomethacin (Sigma), 10 µg/ml of insulin (Sigma) and 0.5 mM 3-isobutyl-1-methylxanthine (Sigma).
Medium B: DMEM (BE12-614F Cambrex, Biowhitaker), 10% FBS (5253 Linus Cultek), 2 mM L-glutamine (Gibco), 100 U/mL of penicillin/streptomycin (Gibco) and 10 µg/ml of insulin (Sigma).

### ○ Chondrogenic Differentiation

The induction of differentiation into chondrocytes was performed following a protocol already established for subcutaneous adipose tissue-derived stem cells and for other cell types (Zuk et al., 2002, cited above). The differentiation was induced by means of the combination of changing the medium and subjecting the cells to a low oxygen concentration.
Preinduction: The clones were centrifuged in conical bottom 96-well plates, and the cell agglomerates were left growing for 24 hours in DMEM with 1% FBS.
Induction: The clones were maintained for 20 days in a specific medium, changing the medium every 3 days, with medium made up of DMEM (BE12-614F Cambrex, Biowhitaker), 1% FBS (5253 Linus Cultek), 2 mM L-glutamine (Gibco), 100 U/mL of penicillin/streptomycin (Gibco), 6.25 µg/ml of insulin (Sigma), 10 ng/mL of TGFB-1 (R&D) and 50 nM ascorbic acid-2-phosphate (Sigma).

### Staining of epi-ADSC cells differentiated into osteocytes, adipocytes and chondrocytes

Different staining protocols suitable for each of the differentiations were used:

### ○ Staining for osteogenic differentiation:

The cells were fixed with 50% ethanol for 15 minutes at 4°C. The staining was performed with 1% Alizarin Red in distilled water (pH = 4.1) at room temperature under stirring for 45 minutes. The colonies that have differentiated and, therefore, have generated calcium around them, are stained an intense red.

### ○ Staining for adipogenic differentiation:

The cells were fixed with a 1:10 dilution of 37% formaldehyde in PBS (pH 7.4) for 20 minutes at room temperature. The staining was performed with Oil Red (0.3 g of Oil Red in 100 ml of isopropanol, 1:2 dilution in distilled water) and incubation at room temperature for 20 minutes. The lipids accumulated in the vacuoles of the cells that have differentiated are stained red.

### ○ Staining for chondrogenic differentiation:

The cells were fixed with a dilution of 4% formaldehyde for 1 hour at room temperature. The staining was performed with a 0.1% dilution of Alcian blue in "acid alcohol" (70% ethanol, 1% HCl in distilled water) incubating at room temperature for 1 hour. After washing, the stain is fixed again with 4% PFA. The differentiated cells are stained blue.

### Results

The analysis of the results of the staining specific for the adipogenic, osteogenic and chondrogenic lineages shows that the stem cells derived from fatty tissue of the heart, particularly from the epicardial area (epi-ADSC), are stained significantly worse than subcutaneous adipose tissue-derived stem cells (sub-ADSC). The appearance of an intense color is indicative of a greater degree of differentiation, therefore it can be concluded that the cardiac adipose tissue-derived stem cells (epi-ADSC) have a lower capability for differentiation into adipocytes, osteocytes and chondrocytes with respect to the subcutaneous adipose tissue-derived stem cells (sub-ADSC).

### EXAMPLE 6

### Isolation and characterization of human cardiac adipose tissue-derived stem cells (ADSC)

### 1. Materials and methods

### 1.1 Collection of cardiac adipose tissue and cell culture

Cardiac adipose tissue biopsy samples were obtained from patients who were subjected to heart surgery before starting a cardiopulmonary bypass. Epicardial adipose tissue biopsy samples (of about 0.5 to 1.0 g on average) were taken from close to the heart and around the aorta. Cardiac adipose tissue biopsies from 117 patients (age: 67.5 ± 9.2 years) who provided their informed consent were used to conduct this study. The study was approved by the Ethics Committee of the Santa Creu i Sant Pau Hospital.

The samples were processed and isolated such as described by Martinez-Estrada, O.M., et al., Human adipose tissue as a source of Flk-1+ cells: new method of differentiation and expansion. Cardiovasc Res 65, 328-33 (2005). The adhered cells were grown until subconfluence in α-MEM medium supplemented with 10% FBS and 1% penicillin-streptomycin (Gibco Invitrogen Corp., Grand Island, NY, USA) and were cultivated in usual conditions.

### 1.2 Clonogenic assay

A clonogenic assay was performed following the protocol described by McFarland [McFarland, D.C. Preparation of pure cell cultures by cloning. Methods Cell Sci 22, 63-6 (2000)]. Briefly, cells were seeded in plates at a density of 400 cells/100 cm² and individual clones were left to develop until they reached several millimeters (mm) in diameter. Then the medium was removed and cloning rings were placed to surround the colony. α-MEM complete medium supplemented with 20% FBS and 1% penicillin-streptomycin was added in the cloning rings and the plates were cultivated in usual conditions.

### 1.3 Evaluation of the teratoma

To evaluate the teratogenic potential of the human cardiac ADSCs, subcutaneous and intramuscular injections (1.5 x 10⁶ cells) were performed in 4 SCID mice (CB17xC57Bl/6) (30 g, Charles River Laboratories Inc. Wilmington, MA, USA) 12 weeks of age. The animals were examined weekly to evaluate tumor formation. Four months after the injection of cells, the skin, skeletal muscle, liver and spleen were collected and processed for their histological analysis.

### 1.4 Adipogenic and osteogenic differentiation assay

Expanded primary cell cultures were subjected to assay to determine the adipogenic and osteogenic potential. Differentiation and alizarin red S staining assays as has been previously described [Phillis, B.D., et al. Modification of d-amphetamine-induced responses by baclofen in rats. Psychopharmacology (Berl) 153, 277-84 (2001); Roura, S. et al. Effect of aging on the pluripotential capability of human CD105+ mesenchymal stem cells. Eur J Heart Fail 8, 555-63 (2006)] were performed.

### 1.5 Flow cytometry

The cells were collected in passage two and were subjected to immunostaining with monoclonal antibodies specific for CD105 (Serotec), CD44, CD166, CD29, CD90, CD117, CD106, CD34, CD45, CD14, CD133 and VEGFR2 (BD Pharmingen). The flow cytometry levels of each antigen were defined by means of the ratio between the specific antibody and the IgG isotype control (Caltag Laboratories, Burlingame, CA, USA) (1 = without difference). A Coulter EPICS XL flow cytometry (Beckman Coulter, Miami, FL, USA) was used to acquire all the data and the analyses were performed using the Expo32 program (Beckman Coulter).

### 1.6 Immunosuppression assay

To analyze the effect of the cardiac ADSCs on peripheral blood lymphocyte (PBL) proliferation, cardiac ADSCs with 2 x 10⁵ PBL and in the presence or absence of the suitable stimulus (PHA 5 µg/ml) were seeded in 5 x 10³ plates. Subcutaneous ADSC of donor L100605 (CMDL) were seeded in plates as a control for immunosuppression. After 4 days of stimulation, BrdU was added to the media for 24 hours and proliferation was determined by means of ELISA following the manufacturer's instructions (Cell proliferation ELISA BrdU, Roche). The experiment was performed in triplicate. The data is shown with respect to PBL proliferation without progenitor cells.

### 1.7 Analysis of GeneChip expression

The total RNA of cardiac ADSCs was isolated in passage 2 of 4 different patients using the QuickPrep total RNA extraction kit (Amersham, Freiburg, Germany) according to the manufacturer's instructions. cRNA was prepared from total RNA, hybridized with Affymetrix HG-U133 Plus 2.0 chips and analyzed to determine the genes expressed differentially. The GeneChip microarray was processed by the *Grupo de Genómica Funcional* (Functional Genomic Group) in the *Instituto de Investigación en Biomedicina* (Institute of Biomedical Research) (Barcelona, Spain) according to the manufacturers' protocols (Affymetrix, Santa Clara, CA) as previously described [Virtaneva, K. et al. Expression profiling reveals fundamental biological differences in acute myeloid leukemia with isolated trisomy 8 and normal cytogenetics. Proc Natl Acad Sci U S A 98, 1124-9 (2001)]. The expression signals were scanned in a Hewlett-Packard GeneArray Scanner.

The statistical analysis of the data was performed using R. First, the data was normalized without processing using the gcRMA algorithm implemented in R, then the probes were filtered using FLUSH [Calza, S. et al. Filtering genes to improve sensitivity in oligonucleotide microarray data analysis. Nucleic Acids Res 35, e102 (2007)] and the relevant changes were extracted using GenePattern [Reich, M. et al. GenePattern 2.0. Nat Genet 38, 500-1 (2006)]. The obtained results were compared with those of a published array on non-differentiated subcutaneous adipose tissue-derived cells [Tchkonia, T. et al. Identification of depot-specific human fat cell progenitors through distinct expression profiles and developmental gene patterns. Am J Physiol Endocrinol Metab 292, E298-307 (2007)] obtained from the GEO database (http://www.ncbi.nlm.nih.gov/geo/; registration ID: GDS2366). To that end, each set of in the array was classified (both studies were performed in the same platform) with a percentage range strategy, and then this percentage range was compared between these 2 studies. The use of a percentage range instead of intensity values allowed preventing any systematic bias which may have been present in any hybridization.

### 1.8 Quantitative real-time RT-PCR

The total RNA of cardiac and subcutaneous ADSC was isolated such as previously explained. cDNA was synthesized from 2 mg of total RNA using random hexamers (Qiagen) and the ScriptTM One-Step RT-PCR kit (BioRad Laboratories) according to the manufacturer's protocol. The details of the quantitative real-time RT-PCR protocol are described below.

Briefly, amplifications were performed by means of PCR with 2 ml of cDNA at a final volume of 50 µl which contained 25 ml of TaqMan 2X Universal PCR Master Mix and 2 ml of each probe/primer marked with FAM acquired from Applied Biosystems (Foster City, CA, USA): GATA4 (Hs00171403_ml), connexin 43 (Cx43) (Hs00748445_s1), SERCA2 (Hs00544877_m1), cardiac troponin I (cTn-I) (Hs00165957_m1), sarcomeric α-actinin (Hs00241650_m1), β-myosin heavy chain (β-MHC) (Hs00165276_ml), VCAM-1 (Hs00365486_m1), von Willebrand factor (vWF) (Hs00169795_m1), VE-cadherin (Hs00174344_m1), CD34 (Hs00990732_m1), EGR-3 (Hs00231780_m1), CD102 (Hs00168384_m1), CD36 (Hs00169627_m1), VEGF-A (Hs00173626_m1), EGR-1 (Hs00152928_m1), CD31 (Hs00169777_m1), SDF-1 (Hs00930455_m1), CXCR-4 (Hs00237052_m1) and glyceraldehyde-3-phosphate dehydrogenase (GAPDH) (Hs99999905_m1). The data was collected and analyzed in the ABI Prism 7000 (ABI) sequence detection system. Each sample was analyzed in duplicate. The Δ threshold cycle method (Ct) was used (Ct is the PCR cycle in which an increase in the indicator fluorescence above the initial level is detected for the first time) to calculate the relative quantification of the expression of each gene, using GAPDH as an endogenous reference as has already been described [Pfaffl, M.W. A new mathematical model for relative quantification in real-time RT-PCR. Nucleic Acids Res 29, e45 (2001)].

### 1.9 Protein extraction and Western blot

Protein extractions were obtained following an already described method [Roura, S. et al. Idiopathic dilated cardiomyopathy exhibits defective vascularization and vessel formation. Eur J Heart Fail 9, 995-1002 (2007)]. The protein levels were normalized by means of the Bio-Rad DC protein assay (Bio-Rad) with bovine serum albumin (BSA), and the samples were separated in 5-10% SDS-PAGE gels. The proteins were transferred on nitrocellulose membranes (Bio-Rad), which were examined with monoclonal antibodies (AcM) specific for GATA4 (1:500), SERCA2 (1:100), α-actin (1:300), cTnI (1:300) (Santa Cruz Biotech.), Cx43 (1:500) (BD Transduction, Lexington, KY, USA), β-MHC (1:10) (Chemicon, Temecula, CA, USA) and sarcomeric α-actin (1:500) (Sigma, St. Louis, MO, USA) respectively. An enhanced chemiluminescence detection system (Amersham Biosciences) was used to view the protein bands.

### 1.10 Immunocytofluorescence staining

The cells were permeabilized, blocked in 5% normal goat serum for 30 minutes and marked with anti-GATA4, anti-SERCA2, anti-cTnI (2 µg/ml) (Santa Cruz Biotechnology), anti-α sarcomeric actin (dilution 1:500) (Sigma), anti-β-MHC (without diluting) (Chemicon) and anti-Cx43 (2.5 µg/ml) (BD Transduction) antibodies for 1 hour at room temperature. Secondary antibodies conjugated with Alexa Fluor 488 and Alexa Fluor 568 (5 µg/ml) (Molecular Probes) were applied and the signals were viewed with confocal laser scanning microscopy (Leica TCS SP5).

### 1.11 Cardiac ADSCs coculture

Cardiac ADSCs were marked with eGFP by means of viral transduction as previously described [Gandia, C. et al. Human dental pulp stem cells improve left ventricular function, induce angiogenesis, and reduce infarct size in rats with acute myocardial infarction. Stem Cells 26, 638-45 (2008)]. Cardiomyocytes of neonatal rats were isolated by means of enzymatic dispersion from newborn rats (1-3 days of age) as previously described [Fukuhara, S. et al. Direct cell-cell interaction of cardiomyocytes is key for bone marrow stromal cells to go into cardiac lineage in vitro. J Thorac Cardiovasc Surg 125, 1470-80 (2003)]. The cardiomyocytes were maintained on plates coated with 2% gelatin at a density of 5 x 10⁴ cells/cm² in 4:1 DMEM:M-199 (Sigma) supplemented with 5% FBS, 10% horse serum (Invitrogen), 1% penicillin-streptomycin and 100 µM cytosine β-D-arabinofuranoside (Sigma) for experiments. Then the eGFP+ ADSC and the neonatal cardiomyocytes were mixed at a ratio of 1:25 and they were seeded at a cell density of 5 x 10⁴ cells/cm². The cells were cultivated together (cocultivated) uninterruptedly at 37°C in 5% CO₂ in air for 30 days.

### 1.12 Endothelial differentiation assay

Cardiac ADSCs were expanded and the endothelial differentiation was analyzed as previously described [Heydarkhan-Hagvall, S. et al. Human adipose stem cells: a potential cell source for cardiovascular tissue engineering. Cells Tissues Organs 187, 263-74 (2008); Liu, J.W. et al. Characterization of endothelial-like cells derived from human mesenchymal stem cells. J Thromb Haemost 5, 826-34 (2007)]. The incorporation of Dil-Ac-LDL (10 µg/ml, Biomedical Technologies) was used to evaluate the endothelial differentiation.

### 1.13 In vitro formation of vascular structures

To induce tubulogenesis, cardiac ADSCs were seeded at a density of 26,000 cells per cm² on plates coated with 1% ECMatrix^{™} (Chemicon International) and tubule formation was checked at 2, 4 and 7 hours with biotinylated GSLI isolectin B4 (*Griffonia Simplicifolia* I B4 lectin) (Vector Labs). Streptavidin conjugated with Alexa Fluor 568 (5 µg/ml) (Molecular Probes) was used to detect the marked cells.

### 1.14 Vasculogenesis potential of cardiac ADSCs

Conditioned medium was obtained from 10,000 cells/cm² in passage 2 cultivated with normoxia (21% O₂), moderate hypoxia (5% O₂) and severe hypoxia (1% O₂) for 24 hour. The angiogenic cytokine concentration was analyzed using a multiplex immunoassay (Procarta Cytokine Assay Kit, Panomics). The analyzed cytokines in conditioned medium were IL-1β, IL-6, TNF-α, VEGF, PDGF_{BB} and bFGF. The results were expressed as mean ± s.d. (pg) of factor secreted by 10⁶ cells at the time of the collection of the medium.

### 2. Results

### 2.1 Isolation and characterization of cardiac ADSCs

Populations of stem (progenitor) cells were satisfactorily isolated in all the cardiac adipose tissue samples of the patients who were subjected to heart surgery, were expanded in cultures in single layer and characterized (Figure 6). A few elongated cells similar to joined fibroblasts appeared after 3 days in culture in the described conditions (Figure 6b).

These cells are clonogenic, have a duplication time (T_{d}) of approximately 5 days and do not induce teratoma formation in SCID mice (data not shown). It is interesting to observe that the culture of the epicardial adipose tissue-derived stem cells (epi-ADSC) with adipogenic and osteogenic media did not result in the intracellular accumulation of lipid drops nor in the extracellular calcium deposition (Figure 7a); whereas, in contrast, the subcutaneous adipose tissue-derived stem cells (sub-ADSC) easily acquired an adipogenic lineage (Figure 7b).

The surface marker profile was examined to immunophenotypically characterize the isolated cardiac ADSCs. More than 90% of the cells expressed a mesenchymal stem cell (MSC) type pattern. Said cells were strongly positive for CD105, CD44, CD166, CD29 and CD90, and weakly positive or negative for CD106, CD117, CD34, CD45, CD14 and CD133 and VEGFR2 (Figure 6c).

Additionally, the cardiac ADSCs could partially inhibit peripheral blood lymphocyte proliferation (a 42% proliferation reduction), which indicates a moderate immunosuppressive capability of the cardiac ADSCs.

### 2.2 Cardiomyogenic lineage differentiation of the cardiac ADSCs

A microarray GeneChip analysis was performed to analyze the gene expression profile of the cardiac ADSCs. The results were compared with the gene expression of non-differentiated subcutaneous adipose tissue-derived cells obtained from the GEO database. Out of the approximately 22,000 genes examined, a different expression of some cardiac markers within the cardiac ADSCs were found in comparison with the subcutaneous adipose tissue-derived stem cells (Table 3).

Using quantitative real-time RT-PCR (Figure 8), a very high expression of the GATA4 transcription factor and of connexin 43 (Cx43), a protein responsible for the electrochemical coupling between adjacent cardiomyocytes, was detected in the cardiac ADSCs in comparison with the isolated subcutaneous adipose tissue stem cells. The transcript levels for SERCA2, cTnI, sarcomeric α-actinin and β-MHC were similar in both cell populations.

At the protein level, in initial culture media, the cardiac ADSCs expressed β-MHC, SERCA2, sarcomeric α-actin, Cx43 and GATA4 (Figure 9a) and traces of Tbx5 (data not shown). The results were confirmed by means of Western blot (Figure 9a) and immunofluorescence (Figure 9b-9e). As can be seen, the β-MHC fibers already express a defined cytoplasmic distribution (Figure 9b). In contrast, the subcutaneous ADSC showed an absence of β-MHC, Cx43, cTnI and GATA4, and a lower expression of sarcomeric α-actin (Figure 9a).

The cocultures of human cardiac ADSCs and neonatal rat cardiomyocytes showed the cardiogenic potential of the analyzed human cells. The intensity and disposition of β-MHC (Figure 10c), sarcomeric α-actin (Figure 10n), Cx43 (Figure 10m), SERCA2 (Figure 10q) and GATA4 (Figure 10r) were enhanced in coculture and were comparable to those observed in the neonatal cardiomyocytes. More importantly, the coculture stimulated expression of troponin I, an important sarcomeric protein not observed in the non-stimulated culture (Figures 10b, 10f and 10j). The arrangement in the troponin I cytoplasm also resembled the sub-cellular sarcomeric organization observed in cardiomyocytes in culture.

### 2.3 Differentiation of the cardiac ADSCs in endothelial lineage in culture

As previously mentioned, the GeneChip microarray analysis performed in cardiac ADSCs and its comparison with non-differentiated subcutaneous adipose tissue-derived cells showed an expression with a greater percentage range of proangiogenic genes in the cardiac ADSCs. Additionally, the expression of angiogenesis inhibitors was predominantly low (Table 4).

To determine the endothelial lineage potential of the human cardiac ADSCs, cells were cultivated with EGM-2 differentiation medium. The comparison of the endothelial transcript levels in treated and non-treated (control) cells showed an increase of the expression of the endothelial markers CD34, VEGF-α, VCAM-1, VE-cadherin, ERG-1, ERG-3, CD31 and SDF-1 (Figure 11). It is interesting to observe that the SDF-1 factor, which benefits the migration of endothelial progenitor cells and enhances vasculogenesis, experienced an increase in the expression of 120 fold [Yamaguchi, J. et al. Stromal cell-derived factor-1 effects on ex vivo expanded endothelial progenitor cell recruitment for ischemic neovascularization. Circulation 107, 1322-8 (2003); Zhang, M. et al. SDF-1 expression by mesenchymal stem cells results in trophic support of cardiac myocytes after myocardial infarction. Faseb J 21, 3197-207 (2007)], whereas CD31, ERG1 and ERG3 increased more than 15, 10 and 16 fold, respectively. Furthermore, the endothelial differentiation of these cells was also demonstrated by means of the rapid incorporation and metabolization of DiI-Ac-LDL [Voyta, J.C. et al. Identification and isolation of endothelial cells based on their increased uptake of acetylated-low density lipoprotein. J Cell Biol 99, 2034-40 (1984)] (Figure 11b).

A functional angiogenic assay was additionally performed to verify the capability of the cardiac ADSCs for differentiating into endothelial lineage. After cultivating the cells in a Matrigel coating and usual conditions, tubular structures were formed. These structures were quickly developed to form a tubular network, growing in its organization and in its diameter (Figures 11c-11e). They were also stained to detect the specific endothelial marker GSLI isolectin B4 (Figures 11f and 11g).

### 2.4 Cardiac ADSCs secrete proangiogenic factors

The *in vitro* secretion of proangiogenic factors was analyzed in hypoxia conditions to test if the cardiac ADSCs could secrete these factors in host tissue when they are injected in the myocardial ischemia and thus enhance vessel formation. In normoxia, the cells secreted significant amounts of IL-6 (53.677 ± 24.613 pg/ml/10⁶ cells) and VEGF (3.201 ± 1.011 pg/ml/10⁶ cells), and slight amounts of bFGF (161.0 ± 31.2 pg/ml/10⁶ cells) and TNF-α (59.1 ± 16.0 pg/ml/10⁶ cells). Expression of IL-1β or of PDGF_{BB} was not detected. In moderate hypoxia (5% O₂) and severe hypoxia (1% O₂), there was a considerable increase of the secretion of VEGF (92%; p=0.04), and the IL-6, bFGF and TNF-α concentrations increased by approximately 20%. The IL-1β and PDGF_{BB} levels remained undetectable.

### EXAMPLE 7

### Cardiac ADSCs transplantation improves cardiac function after myocardial infarction

### 1. Materials and methods

### 1.1 Model of myocardial infarction and cell transplantation

### 1.1.1Rats

A total of 16 male nude rats (200-250 g; *NIH-Foxn1^{rnu},* Charles River Laboratories Inc. Willmington, MA, USA) was used for the study. The left coronary artery was ligated as previously described [Gandia, C. et al. Human dental pulp stem cells improve left ventricular function, induce angiogenesis, and reduce infarct size in rats with acute myocardial infarction. Stem Cells 26, 638-45 (2008)]. The rats were intubated and anesthetized with an O₂/Sevorane mixture and mechanically ventilated (Harvard Apparatus model 683 small animal ventilator), and after the thoracotomy, an acute myocardial infarction was induced by means of permanent ligation of the LAD coronary artery with 6-0 prolene. The incision was closed with a 3-0 silk suture. After a week, the rats were anesthetized and opened up again by means of median sternotomy to perform the intramyocardial transplantation (10⁶ cardiac GFP-ADSC cells suspended in saline or an equal volume of saline) in 5 injections of 5 µl of volume in 5 points of the border area of the infarction with a Hamilton syringe.

After approximately 30 days from the injection of cells, the hearts were stopped in diastole with a stop solution (68.4 mM NaCl, 59 mM KCl, 11.1 mM glucose, 1.9 mM NaHCO₃, 29.7 mM BDM (2,3-butanedione-monoxime), 1,000 U heparin), they were sectioned, fixed, cryopreserved in 30% sucrose in PBS, embedded in OCT (Sakura, Torrance, California, USA) and instantly frozen in isopentane cooled with liquid nitrogen. Blocks of tissue were stored at -80°C until they were sectioned.

In all processes the standards from the Guide for the Care and Use of Laboratory Animals of the Institute of Laboratory Animal Research (NIH Pub. No. 86-23, revised in 1996) were used.

### 1.1.2 Echocardiography

To evaluate cardiac function, a transthoracic echocardiography was performed in rats as previously described [Friedrich, J. et al. 31P nuclear magnetic resonance spectroscopic imaging of regions of remodeled myocardium in the infarcted rat heart. Circulation 92, 3527-38 (1995)]. An echocardiographic system (General Electric) equipped with a 10 MHz linear network transducer was used, and measurements were taken at the initial level (1 day before the infarction) and 15 and 30 days after the cell transplantation. Central papillary two-dimensional (2-D) M-mode echocardiographs were performed in the parasternal short axis view. The functional parameters were calculated on five consecutive cycles cardiac using conventional methods [Litwin, S.E., Katz, S.E., Morgan, J.P. & Douglas, P.S. Serial echocardiographic assessment of left ventricular geometry and function after large myocardial infarction in the rat. Circulation 89, 345-54 (1994)].

The dimensions of the anterior and posterior wall (AW and PW) in diastole and systole, the dimensions of the LV in the end diastole (LVDd) and end systole (LVDd), end diastolic area (EDA) and end systolic area (ESA) were quantified. The changes in AW and PW were calculated as (AWs/AWd-1)x100 and (PWs/PWd-1)x100, respectively. The fractional shortening (FS) was calculated as [(LVDd-LVDs)/LVDd]x100 and the ejection fraction (EF) as [(LVEDV-LVESV)/LVEDV]x100.

### 1.2 Morphometry

### 1.2.1 Rats

Rat hearts were cut cross-wise into three segments: apex, middle (containing the ligation) and base. For the measurements, 10 µm thick cryosections (6 sections, separated 200 µm) of the middle segment were serially stained with Masson's trichrome and the morphometric parameters were determined using image analysis software (ImageJ, NIH). The infarction size was measured as a percentage of the average scar area in the total LV wall surface. The thickness of the infarction was calculated by means of the sum of the partial endocardial infarction areas [Takagawa, J. et al. Myocardial infarct size measurement in the mouse chronic infarction model: comparison of area- and length-based approaches. J Appl Physiol 102, 2104-11 (2007)]. All the sections were examined blindly and photographed using a Leica stereoscope (Leica TL RCI).

### 1.2.2 Immunofluorescent histology

Double immunostains were performed in cryosections of rat heart. The tissues were incubated with the primary antibodies for CD31 (1:25) (Abcam) and sarcomeric a-actin (dilution 1:500) (Sigma) or cTnI (2 µg/ml) (Santa Cruz Biotechnology). For the immunohistological detection of human cells in rat hearts, the tissue sections were incubated with anti-human nuclear antigen antibody (HNA, Chemicon). Secondary antibodies conjugated with Alexa Fluor 488, Alexa Fluor 568 and Alexa Fluor 647 (5 µg/ml) (Molecular Probes) were used. The sections were counterstained with 4,6-diamidino-2-phenylyndole (DAPI, Sigma) and were analyzed with Leica TCS SP5.

### 1.2.3 Capillary density

To determine the capillary density in border areas of the infarction and in distal myocardium of the infarction, heart sections were stained using biotinylated GSLI isolectin B4 (Vector Labs). Streptavidin conjugated with Alexa Fluor 568 was used as a detection system. The capillaries were counted in at least 12 randomly selected fields (6 border areas + 6 distal areas) in 4 control animals and 5 treated animals. The results were expressed as a mean capillary number per surface of tissue in mm². Two independent observers blind to the treatment analyzed the control group and treated group with a correlation coefficient between classes (CHF) of 0.821 in the border area (p < 0.001) and 0.743 in the distal area (p < 0.001). Taking into consideration that the values obtained by the investigators were similar, the final result of each point was the mean ± SEM.

### 1.2.4 Statistical analysis

All the sections were examined blindly, counted and reviewed by two of the investigators. The statistical significance of the FS, EF and AWT data was estimated by means of analysis of variance (two-way ANOVA) for multiple comparison between groups and Greenhouse's correction was applied. The echocardiographic parameters during the MI and after 30 days (Table 5) and the morphometric values between the control group and with cells using the t-test were all compared. The results were presented as mean ± SD. The differences in the capillary density were also compared between the control groups and with cells in the border and distal areas using the t-test. It was considered that the values of p <0.05 were significant between groups. Descriptive statistics were performed with SPSS.

### 2. Results

### 2.1 Cardiac ADSCs transplantation improves cardiac function after myocardial infarction

Echocardiographic parameters of control and cardiac ADSCs groups were obtained in the initial level, after a myocardial infarction (MI), and up to 30 days after the injection of cells in nude rats (Table 5). In the initial level and after the MI, the values of the echocardiographic parameters analyzed were similar in treated and non-treated animals, which indicates comparable levels of tissue injury. After the MI, a significant functional improvement was detected in the treated group between the MI and at 30 days (Table 5). The evolution of the control group showed a progressive deterioration of the cardiac function parameters due to the remodeling of the left ventricle (LV), determined by means of the diameters and the end diastolic and end systolic areas of the LV (Table 5). Significant differences were found between the control group and the group treated with cells in the fractional shortening (p=0.024) and the ejection fraction (p=0.003) (Figure 12a and 12b) using the two-way ANOVA system. Furthermore, the anterior wall was significantly thicker after 30 days of treatment with cardiac ADSCs (p=0.014) (Figure 12c). The video recording of cardiac mobility showed an improvement in the contractility in the cardiac ADSCs group but not in the control group.

### 2.2 Cardiac ADSCs transplantation reduces the size of the infarction

Cross sections stained with Masson's trichrome were used to measure the morphometric parameters in MI rat models. A moderate scar was developed after ligation of the LAD (approximately 20% of the LV area), and the beneficial effect of the cardiac ADSCs was very considerable. The percentage of the fibrous scar tissue area was 75% lower in treated animals in comparison with the control animals and the LV wall was 45% thicker in treated animals. Therefore, the cardiac ADSCs effectively reduce the size of the infarction measured by means of histopathology after 30 days from the injection of cardiac ADSCs.

### 2.3 Human cardiac ADSCs graft and in vivo differentiation

The human cardiac ADSCs graph was analyzed by tracing the eGPF epifluorescence and the human origin was demonstrated by means of HNA (human nuclear antigen) detection (red signal) (Figure 14a). The specific signal of eGFP was additionally confirmed by means of spectral analysis of stain separation (lambda exploration) (Figure 15). It is interesting to observe that the cells were uniformly located inside the scar area of the tissue independently of the injection being directed at the border area of the infarction, which indicates the capability of the cells to migrate towards where they may especially be needed (Figure 14b). For the purpose of analyzing the cardiac and endothelial differentiation level of the injected cells and their graft in the host tissue, double immunostaining for cardiac markers (sarcomeric α-actinin and troponin I) and endothelial markers (CD31) was performed. The cardiac eGFP+-ADSC again showed expression of troponin I (Figure 14b) and were positive for sarcomeric α-actinin (Figure 14c). Expression of CD31 was also observed in the cardiac eGPF+-ADSCs, which were arranged throughout the tissue forming tubular structures, which suggests that these cells contribute to the formation of new vessels (Figure 14d).

### 2.4 Increase of the capillary density due to cardiac ADSCs

Finally, GSLI isolectin B4 was used to evaluate the capillary density in the border areas of the fibrotic tissue and a time after the cardiac ADSCs transplantation. As illustrated in Figure 16c, the capillary density in the border area was 1.6 times greater in the animals that received cardiac ADSCs than in the control animals (p=0.003) (Figures 16a and 16b) and a trend towards the increase of the capillary density in the distal areas (p=0.057) was also found. The presence of blood cells in their lumen indicates that these vessels were functional.

### 3. Discussion

The results shown in Examples 1-7 attached to this description clearly show that a novel population of cardiac adipose tissue-derived adult stem cells (cardiac ADSCs) has been identified and characterized. Said cardiac ADSCs express surface markers similar to mesenchymal stem cells (MSC) and preserve clonogenic capability and, although they are from adipose tissue, they do not differentiate into adipocytes like MSC do, which indicates lower plasticity and a greater assigned condition. Furthermore, the cardiac ADSCs express several essential cardiomyogenic markers, such as GATA4, Cx43, β-MHC, sarcomeric α-actinin, or SERCA2. The comparison of the gene expression and of cardiomyogenic proteins in cardiac and subcutaneous ADSC showed that the expression of these proteins was considerably greater in the cardiac ADSCs. These results suggest that the novel cardiac ADSCs, despite residing in an adipocytic environment, can have a function in cardiac homeostasis. The fat surrounding the heart can also function as a reservation of cells for renewing myocardial tissue; nevertheless, the large amount of myocardium at risk which is produced after an infarction exceeds the homeostatic capability of tissue repair.

When the cardiac ADSCs was under the influence of neonatal rat cardiomyocytes, the expression of the cardiomyogenic markers was significantly regulated upwards (α-actinin sarcomeric, β-MHC, SERCA2) or activated *de novo* (troponin I). Earlier studies demonstrated that the mechanism by means of which the neonatal cardiomyocytes stimulated cardiac differentiation could be the secretion of differentiation factors, interactions between cells and electric and mechanical stimulations. Furthermore, the cultivation of cardiac ADSCs in Matrigel or in an endothelial differentiation medium led to the formation of tubular structures, to the incorporation of Dil-Ac-LDL and to the enhancement of the expression of endothelial markers. *In vivo* experiments also demonstrated the capability of the cardiac ADSCs to differentiate into cardiac cell lineages. When they were transplanted into infarcted myocardium, it was observed that said cardiac ADSCs expressed sarcomeric α-actinin, cTnI and CD31 and were effectively grafted in the myocardium. The cell interactions together with the electric and mechanical influence of tissue stimulated their differentiation. The cell transplantation resulted in an improved cardiac function, the ejection fraction, the fractional shortening and the LV wall thickness in animals treated with cells being significantly increased. The beneficial effects of the cardiac ADSCs were equal to or greater than those observed by bone marrow cells [Agbulut, O. et al. Comparison of human skeletal myoblasts and bone marrow-derived CD133+ progenitors for the repair of infarcted myocardium. J Am Coll Cardiol 44, 458-63 (2004)] and adult cardiac stem cells [Beltrami, A.P. et al. Adult cardiac stem cells are multipotent and support myocardial regeneration. Cell 114, 763-76 (2003)] with an improvement of the ejection fraction of 7 and the 9.7%, respectively, or umbilical cord blood mononuclear cells [Henning, R.J. et al. Human umbilical cord blood mononuclear cells for the acute myocardial infarction treatment. Cell Transplant 13, 729-39 (2004)] which showed a 27% reduction of the ejection fraction when they were transplanted in infarcted myocardium.

For the purpose of determining if the cardiac ADSCs could survive in ischemic tissue and benefit angiogenesis, cells were cultivated in moderate and severe hypoxic conditions, and the secretion of proangiogenic factors into the medium was analyzed. The results showed an increase in the VEGF, TNF-β, bFGF and IL-6 levels in comparison with normoxia. It has been reported that when a single angiogenic factor was supplied to patients with arteriosclerosis, the neovascularization response was only moderate. A possible explanation of this result could be that the formation of the vessel network and its expansion is a process which requires the action of multiple factors acting synergistically. Therefore, the secretion of a combination of proangiogenic factors by the cardiac ADSCs in baseline conditions and their increase in hypoxic situations makes these cells potentially useful for their injection in myocardial ischemia. In fact, a clear increase in the capillary density was observed after the cardiac ADSCs transplantation in the infarcted heart. All this globally suggests that these cells (cardiac ADSCs) can have a paracrine effect on the myocardial ischemia, benefiting the formation of new vessels. A large number of capillaries improves the oxygen limitations in the myocardium, thus benefiting such significant reduction in the sizes of infarction, 43% in rats. Furthermore, there are reports which show that an increase of SDF-1 can improve the survival of cardiomyocytes and induce neovascularization after an infarction [Penn, M.S. & Mangi, A.A. Genetic enhancement of stem cell engraftment, survival, and efficacy. Circ Res 102, 1471-82 (2008)]. Therefore, the demonstration of the *in vivo* endothelial lineage of cardiac ADSCs and the considerable overexpression of SDF-1 observed after the *in vitro* endothelial differentiation suggest that SDF-1 could have a cardioprotective effect on the cardiomyocytes as well as an angiogenic effect.

It should be observed that although the cardiac ADSCs were injected in the border area of the infarction, the cells were located within the fibrotic scar. This indicates their capability to migrate from the site of the injection towards ischemic areas, where they may especially be needed. Earlier experiments using other stem cell lineages demonstrate similar results, which were explained by a cell migration response due to the chemotactic effect of VEGF in hypoxic conditions [Gandia, C. et al. Human dental pulp stem cells improve left ventricular function, induce angiogenesis, and reduce infarct size in rats with acute myocardial infarction. Stem Cells 26, 638-45 (2008); Matsushita, K. et al. The role of vascular endothelial growth factor in human dental pulp cells: induction of chemotaxis, proliferation, and differentiation and activation of the AP-1-dependent signaling pathway. J Dent Res 79, 1596-603 (2000)]. In the same order of evidence, a study conducted in chicken embryos showed that cardiac injury is a potent stimulus for the attraction of human umbilical cord blood-derived stem cells [Torre-Perez, N. et al. Migration and differentiation of human umbilical cord stem cells after heart injury in chicken embryos. Stem Cells Dev (2008)].

Cardiac adipose tissue is arranged closely around the heart and accessibility to cardiac ADSCs is a drawback. Although a left lateral thoracotomy could be easily performed to obtain cardiac fat biopsies for the isolation of these cardiac ADSCs, this approach does not seem to be clinically applicable in an emergency situation of a myocardial infarction, although it could be used before the bypass surgery of the coronary arteries in stable ischemic patients. However, the fact that the cardiac ADSCs have an immunosuppressive capability similar to that described for other types of mesenchymal stem cells generates the possibility of a future allogeneic therapeutic use of these cells.

In summary, a novel type of stem (progenitor) cells with inherent endothelial and cardiac potential is described, which cells can differentiate into cardiac cell lineages *in vitro* and *in vivo,* having a beneficial functional and histopathological effect when they are injected in damaged myocardium after an MI. Without the intention of being bound to any theory, it is thought that there may be a double mechanism of remodeling attenuation, in which the cells have the potential of substituting the cardiomyocytes and the endothelial cells lost and also have a paracrine effect on the enhancement of angiogenesis. These properties, together with the capability of immunosuppression and the absence of teratogenicity, make the cardiac ADSCs safe and promising candidates for their future use in cell therapy for regenerating the damaged myocardium.

**Table 3**

| **Expression profiles of cardiac genes in cardiac and subcutaneous ADSCs** | | | | |
|---|---|---|---|---|
| **Gene ID** | **Name of the gene** | **Description** | **Percentile range Cardiac Sub** | |
| 201058_s_at | MRLC-2 | Myosin regulatory light chain 2, smooth muscle isoform (myosin RLC). | 100 | 97 |
| 201667_at | Cx43 | Connexin 43 | 99 | 97 |
| 209186_at | SERCA2 | Sarco/endoplasmic reticulum calcium ATPase 2 (EC 3.6.3.8). | 99 | 96 |
| 202555_s_at | MLCK | Myosin light chain kinase, smooth muscle (EC 2.7.11.18). | 92 | 87 |
| 203216_s_at | Myosin VI | Myosin VI (non-conventional myosin VI). | 91 | 72 |
| 213201_s_at | cTnT | Troponin T, (heart muscle troponin T). | 85 | 53 |
| 206029_at | cAnkyrin | Protein 1 containing the ankyrin repeat domain (cardiac ankyrin repeat protein) (cytokine-inducible nuclear protein) (C-193). | 84 | 26 |
| 203017_s_at | ADIP | Afadin and alpha-actinin binding protein (ADIP) (SSX2 interaction protein). | 79 | 22 |
| 209904_at | TN-C | Troponin C, heart and slow skeletal muscles. | 75 | 21 |
| 205918_at | CAE3/BAE3 | Anion exchange protein 3 (protein similar to neuronal band 3) (member 3 of the solute carrier family 4) (protein similar to the cardiac / cerebral band 3). | 72 | 21 |
| 207961_x_at | MCH-11 | Myosin 11 (myosin heavy chain 11) (myosin heavy chain, smooth muscle isoform). | 62 | 35 |
| 205738_s_at | H-FABP | Heart fatty acid binding protein (H-FABP) (heart-type fatty acid binding protein). | 62 | 34 |
| 217660_at | MHC-14 | Myosin 14 (myosin heavy chain 14) (non-muscular myosin heavy chain IIc) (NMHC II-C). | 58 | 14 |
| 1553131_a_at | GATA4 | GATA-4 transcription factor (GATA binding factor 4). | 56 | 43* |
| 215331_at | MHC-15 | Myosin 15 (myosin heavy chain 15). | 56 | 6 |
| 205940_at | MHC-3 | Myosin 3 (myosin heavy chain 3) (embryonic myosin heavy chain muscle) (SMHCE). | 55 | 36 |
| 203861_s_at | α-Actinin-2 | Alpha-actinin-2 (alpha-actinin skeletal muscle isoform 2). | 52 | 12 |
| 214365_at | Tropomyosin-3 | Tropomyosin alpha-3 chain (tropomyosin 3) (tropomyosin gamma) (hTM5). | 50 | 40 |

| | | | | |
|---|---|---|---|---|
| * Gene ID 205517_at. Variant of different splicing | | | | |

**Table 4**

| **Expression profiles of angiogenic genes in cardiac and subcutaneous ADSCs** | | | | |
|---|---|---|---|---|
| **Gene ID** | **Name of the gene** | **Description** | **Percentile range Cardiac Sub** | |
| 202729_s_at | TGF-b1-BP-1 | Transforming growth factor beta 1 binding protein 1 (TGF-beta1-BP-1) | 99 | 92 |
| 212171_x_at | VEGF-A | Vascular endothelial growth factor A (VEGF-A) precursor | 98 | 75 |
| 209946_at | VEGF-C | Vascular endothelial growth factor C (VEGF-C) precursor | 98 | 93 |
| 218856_at | TNF-a | Tumor necrosis factor receptor superfamily, member 21 (TNFR-related death receptor 6) (death receptor 6) precursor | 97 | 57 |
| 208944_at | TGFR-2 | TGF-beta type 2 receptor (of TGF-beta type II receptor) (TGFR-2) precursor | 97 | 95 |
| 212196_at | IL-6R-b | Interleukin 6 receptor subunit beta (IL-6R-beta) precursor | 94 | 87 |
| 204352_at | α-Actinin-2 | TNF receptor-associated factor 5 (RING finger protein 84) | 94 | 62 |
| 200706_s_at | LPS-induced TNF-a Factor | Lipopolysaccharide-induced tumor necrosis factor alpha factor (LPS-induced TNF-alpha factor) | 94 | 39 |
| 209687_at | SDF-1 | Stromal cell-derived factor 1 (SDF-1) (CXCL12) precursor | 90 | 79 |
| 220407_s_at | TGF-b-2 | Transforming growth factor beta 2 (TGF-beta-2) precursor | 87 | 19 |
| 201693_s_at | EGR-1 | Early growth response protein 1 (EGR-1) | 86 | 18 |
| 205227_at | IL-1R Accessory | Interleukin 1 receptor accessory protein (IL-1 receptor accessory protein) precursor | 85 | 60 |
| 206025_s_at | TSG-6 Precursor | Tumor necrosis factor-inducible protein TSG-6 (TNF-stimulated gene 6 protein) precursor | 84 | 1 |
| 215561_s_at | IL-1R-1 | Interleukin 1 receptor type I (IL-1R-1) precursor | 84 | 71 |
| 209543_s_at | CD34 | CD34 hematopoietic progenitor cell antigen precursor | 82 | 5 |
| 220406_at | TGF-b-2 | Transforming growth factor beta 2 (TGF-beta-2) precursor | 78 | 31 |
| 39402_at | IL-1 b | Interleukin 1 beta (IL-1 beta) precursor (Catabolina) | 76 | 64 |
| 205992_s_at | IL-15 | Interleukin 15 (IL-15) precursor | 74 | 19 |
| 202859_x_at | IL-8 | Interleukin 8 (IL-8) precursor (CXCL8) | 68 | 73 |
| 218658_s_at | IL-17RB | Interleukin 17 receptor B (IL-17 receptor B) precursor | 65 | 5 |
| 204677_at | VE-cadherin | Vascular endothelial cadherin (VE cadherin) (CD144 antigen). | 64 | 25 |
| 206488 s at 206488_s_at | CD36 | Leukocyte-differentiating antigen (CD36) (platelet collagen receptor) (fatty acid translocase) (FAT) | 59 | 39 |
| 208982_at | CD31 | Platelet-endothelial cell adhesion molecule (PECAM-1) precursor (CD31 antigen). | 55 | 55 |
| 217028_at | CXCR-4 | SDF-1 receptor (CXCR-4) | 53 | 46 |
| 202112_at | vWF | Von Willebrand factor (vWF) precursor | 27 | 22 |
| 207539_s_at | IL-4 | Interleukin 4 (IL-4) precursor | 23 | 25 |
| 204912_at | IL-10R-A | Interleukin 10 receptor alpha chain (IL-10R-A) precursor | 20 | 8 |
| 207160_at | IL-12A | Interleukin 12 alpha subunit (IL-12A) precursor | 32 | 23 |
| 206999_at | IL-12R-b2 | Interleukin 12 receptor beta 2 chain (IL-12R-beta2) precursor | 17 | 25 |
| 210904_s_at | IL-13R-a-1 | Interleukin 13 receptor alpha 1 chain (IL-13R-alpha-1) precursor | 64 | 85 |
| 206295_at | IL-18 | Interleukin 18 (IL-18) precursor | 46 | 50 |
| 219115_s_at | IL-20R-a | Interleukin 20 receptor alpha chain (IL-20R-alpha) precursor | 60 | 32 |

| | | | | |
|---|---|---|---|---|
| **Angiogenesis enhancers:** VEGF, TNFa, TGF-b, IL1, IL6, IL7, IL8, IL15, IL23, IL19. **Angiogenesis inhibitors:** IL4, IL10, IL12, IL13, IL18, IL20, IL24. | | | | |

**Table 5**

| **Echocardiographic data** | | | | | | | | |
|---|---|---|---|---|---|---|---|---|
| | **SALINE (n=6)** | | | | **cardiac ADSCs (n=10)** | | | |
| | **MI** | **15d** | **30d** | ***Value of p* of MI against 30d** | **MI** | **15d** | **30d** | ***Value of p* of MI against 30d** |
| **AW diastole (mm)** | 1.10 ± 0.52 | 1.03 ± 0.27 | 1.18 ± 0.24 | n.s. | 0.98 ± 0.36 | 1.17 ± 0.43 | 1.30 ± 0.31 | 0.047 |
| **AW systole (mm)** | 1.50 ± 0.55 | 1.53 +0.43 | 1.57 ± 0.36 | n.s. | 1.38 ± 0.68 | 2.03 ± 0.84 | 2.19 ± 0.60 | 0.011 |
| **PW diastole (mm)** | 1.43 ± 0.29 | 1.47 ± 0.31 | 1.52 ± 0.29 | n.s. | 1.44 ± 0.32 | 1.35 ± 0.32 | 1.67 ± 0.62 | n.s. |
| **PW systole (mm)** | 2.02± 0.40 | 2.25 ± 0.46 | 1.90 ± 0.35 | n.s. | 2.29 ± 0.44 | 2.08 ± 0.27 | 2.49 ± 0.60 | n.s. |
| **LV diastole (mm)** | 6.53 ± 0.43 | 7.12 ± 0.52 | 7.55 ± 0.60 | 0.007 | 7.19 ± 0.58 | 6.97 ± 0.56 | 7.59 ± 0.64 | n.s. |
| **LV systole (mm)** | 4.77 +0.25 | 4.95 ± 0.51 | 5.35 ± 0.75 | n.s. | 5.11 ± 0.45 | 4.57 ± 0.62 | 5.02 ± 0.75 | n.s. |
| **EDA (mm²)** | 0.36 ± 0.08 | 0.40 ± 0.03 | 0.45 ± 0.04 | 0.022 | 0.42 ± 0.05 | 0.45 ± 0.06 | 0.42 ± 0.06 | n.s. |
| **ESA (mm²)** | 0.20 ± 0.03 | 0.20 ± 0.01 | 0.24 ± 0.03 | 0.027 | 0.27 ± 0.05 | 0.25 ± 0.09 | 0.22 ± 0.07 | n.s. |
| **FS (%)** | 28.47 ± 6.10 | 30.52 ± 2.99 | 29.64 ± 7.28 | n.s. | 28.87 ± 4.05 | 34.59 ± 5.18 | 34.13 ± 4.98 | 0.018 |
| **EF (%)** | 60.59 ± 5.84 | 66.25 ± 4.10 | 63.67 ± 8.35 | n.s. | 63.69 ± 6.57 | 71.55 ± 6.38 | 70.98 ± 6.31 | 0.021 |
| **AW Change(%)** | 40.48 ± 15.92 | 47.54 ± 10.84 | 32.08 ± 8.56 | n.s. | 36.74 ± 22.19 | 71.08 ± 34.87 | 68.32 ± 28.77 | 0.013 |

The values are means ± SD; AW indicates anterior wall thickness; PW, posterior wall thickness; LV, internal dimension of the left ventricle; EDA, end diastolic area; ESA, end systolic area final; FAC, fractional area change; FS, fractional shortening; EF, ejection fraction.

## Claims

1. An isolated adult stem cell derived from fatty heart tissue of mammals, constitutively expressing GATA-4 and/or Cx43.

2. The cell according to claim 1, which furthermore constitutively expresses β-MHC.

3. The cell according to any of claims 1 or 2, which furthermore expresses one or more surface markers selected from CD29, CD44, CD59, CD90, CD105 and CD166.

4. The cell according to any of claims 1 to 3, which does not express one, two, three, four, or preferably any of the surface markers CD14, CD34, CD106, CD117 or VEGFR2.

5. The cell according to any of claims 3 or 4, which (i) expresses one, two, three, four, five, or preferably all the surface markers CD29, CD44, CD59, CD90, CD105 and CD166, and (ii) does not express one, two, three, four, or preferably any of the surface markers CD14, CD34, CD106, CD117 or VEGFR2.

6. The cell according to claim 5, selected from
a) a cell which (i) expresses all the surface markers CD29, CD44, CD59, CD90 and CD105, and (ii) does not express any of the surface markers CD14, CD34, CD106 or CD117;
b) a cell which (i) expresses all the surface markers CD29, CD44, CD90, CD105 and CD166, and (ii) does not express any of the surface markers CD14, CD34, CD106, CD117 or VEGFR2; and
c) a cell which (i) expresses all the surface markers CD29, CD44, CD59, CD90, CD105 and CD166, and (ii) does not express any of the surface markers CD14, CD34, CD106, CD117 or VEGFR2.

7. The cell according to any of claims 1 to 6, wherein said fatty heart tissue is fatty epicardial tissue.

8. The cell according to any of claims 1 to 7, wherein said mammal is a human being.

9. The cell according to any of claims 1 to 8, wherein the constitutive expression of GATA-4 and/or Cx43 is maintained stable during its *in vitro* expansion.

10. An isolated adult stem cell derived from fatty heart tissue of mammals, selected from:
(i) an adult stem cell derived from fatty heart tissue which:
a) constitutively expresses GATA-4 and/or Cx43;
b) constitutively expresses β-MHC;
c) expresses all the surface markers CD29, CD44, CD59, CD90 and CD105; and
d) does not express any of the surface markers CD14, CD34, CD106 or CD117;
(ii) an adult stem cell derived from fatty heart tissue which:
a) constitutively expresses GATA-4 and/or Cx43;
b) constitutively expresses β-MHC;
c) expresses all the surface markers CD29, CD44, CD90 and CD105 and CD166; and
d) does not express any of the surface markers CD14, CD34, CD106, CD117 or VEGFR2; and
(ii) an adult stem cell derived from fatty heart tissue which:
a) constitutively expresses GATA-4 and/or Cx43;
b) constitutively expresses β-MHC;
c) expresses all the surface markers CD29, CD44, CD59, CD90 and CD105 and CD166; and
d) does not express any of the surface markers CD14, CD34, CD106, CD117 or VEGFR2.

11. The cell according to claim 10, wherein the constitutive expression of GATA-4 and/or Cx43 is maintained stable during its *in vitro* expansion.

12. An isolated population of adult stem cells derived from fatty heart tissue of mammals, comprising a group of adult stem cells derived from fatty heart tissue of mammals according to any of claims 1 to 11.

13. A process for obtaining a composition comprising adult stem cells derived from fatty heart tissue of mammals constitutively expressing GATA-4 and/or Cx43, comprising:
a) obtaining a cell suspension of a fatty heart tissue sample of a mammal;
b) separating the cells from said cell suspension;
c) cultivating said cells in a culture medium on a solid support under conditions which allow said cells to adhere to said solid support; and
d) recovering the adult stem cells derived from fatty heart tissue of mammals constitutively expressing GATA-4 and/or Cx43.

14. A composition comprising adult stem cells derived from fatty heart tissue of mammals constitutively expressing GATA-4 and/or Cx43 obtainable by means of a process according to claim 13.

15. A method for obtaining differentiated cells comprising cultivating adult stem cells derived from fatty heart tissue of mammals according to any of claims 1 to 11, or obtainable by means of a process according to claim 13, comprising cultivating said stem cells in a suitable specific differentiation medium.

16. A differentiated cell obtainable by means of a process according to claim 15.

17. A composition comprising differentiated cells obtainable by means of a process according to claim 15.

18. A pharmaceutical composition comprising a cell population according to claim 12, or a composition according to claim 14, or a composition according to claim 17, and a pharmaceutically acceptable vehicle.

19. The pharmaceutical composition according to claim 18, for its parenteral administration.

20. A biomaterial comprising a cell population according to claim 12, a composition according to claim 14, a pharmaceutical composition according to any of claims 18 or 19, or a composition according to claim 17.

21. Use of a cell population according to claim 12, or a composition according to claim 14, or a composition according to claim 17, in the preparation of a pharmaceutical composition for cardiac tissue regeneration, or in the preparation of a pharmaceutical composition for the treatment of an ischemic heart disease, or in the preparation of a pharmaceutical composition for the post-myocardial infarction treatment, or for the treatment of congestive heart failure, or in the preparation of a pharmaceutical composition to stimulate angiogenesis.

22. The use according to claim 21, wherein said pharmaceutical composition is administered together with one or more different therapeutic agents simultaneously or sequentially.

23. The use according to any of claims 20 or 21, wherein the cells contained in said cell population are autologous.

24. The use according to any of claims 20 or 21, wherein the cells contained in said cell population are allogeneic.

25. A kit comprising a cell population according to claim 12, or a composition according to claim 14, or a composition according to claim 17.

26. A method for evaluating the *in vitro* cell response to a biological or pharmacological agent, comprising contacting said agent with a cell population according to claim 12, comprising a plurality of stem cells according to any of claims 1 to 11, or a composition according to claim 14, optionally differentiated into a specific cell type, or a composition according to claim 17, and evaluating the effects of said agents on said cell population in culture.

27. The method according to claim 26, wherein said stem cells are differentiated into cardiomyocytes.

28. A process for obtaining growth factors and/or cytokines comprising cultivating adult stem cells derived from fatty heart tissue of mammals according to any of claims 1 to 11, or stem cells obtainable according to the process of claim 13, or differentiated cells obtainable according to the method of claim 15, under conditions suitable for the expression and production of said growth factors and/or cytokines, and, if desired, separating said growth factors and/or cytokines.
